# FASCICULE DE BREVET EUROPEEN

(11) **EP 0 596 802 B1**
(45) Date de publication et mention de la délivrance du brevet: **20.03.1996**
(21) Numéro de dépôt: 93402704.6
(22) Date de dépôt: 04.11.1993
(51) Int. Cl.: C07H 17/00, C07H 17/08, A61K 31/70

(54) **Nouveaux dérivés de l'érythromycine, leur procédé de préparation, leur application comme médicaments**
Erythromycinderivate, ihr Verfahren zur Herstellung und ihre Verwendung als Arzneimitteln
Erythromycin derivatives, their process of preparation and their application as medicaments

(30) Priorité: 05.11.1992 FR 9213320; 02.07.1993 FR 9308109
(43) Date de publication de la demande: 11.05.1994
(73) Titulaire: ROUSSEL UCLAF, F-93230 Romainville (FR)
(72) Inventeur: Agouridas, Constantin, F-94130 Nogent sur Marne (FR); Bonnefoy, Alain, F-93230 Les Lilas (FR); Chantot, Jean-François, F-77410 Gressy en France (FR); Le Martret, Odile, F-75016 Paris (FR); Denis, Alexis, F-75011 Paris (FR)
(74) Mandataire: Vieillefosse, Jean-Claude

(56) Documents cités:
- EP-A- 0 487 411
- WO-A-92/09614
- DE-A- 2 936 865
- CHEMICAL ABSTRACTS, vol. 106, no. 19, 1987, Columbus, Ohio, US; abstract no. 152654m, RENGARAJU ET AL. 'A New Macrolide Antibiotic Kayamycin 10,11-dihydro-5-O-mycaminosyl Narbonolide Produced by Nocardiopsis.' page 363 ;colonne 1 ;
- CHEMICAL ABSTRACTS, vol. 106, no. 21, 1987, Columbus, Ohio, US; abstract no. 176805n, T.FUJIWARA ET AL. '4'-O-(4-O-Acylmycarosyl )mycaminosylnarbonolides.' page 772 ;colonne 2 ;
- ARCHIV DER PHARMAZIE vol. 320, no. 12 , 1987 , WEINHEIM pages 1233 - 1238 K.REHSE ET AL. 'Antiaggregatorische und Anticoagulante Eigenschaften von Oligoaminen, 7. Mitt.: Benzol-1,3,5-trialkylamine.'

## Description

La présente invention concerne de nouveaux dérivés de l'érythromycine, leur procédé de préparation et leur application comme médicaments.

On connaissait déjà des dérivés descladinosylés de l'érythromycine, (voir EPA 0 487 411) possédant des propriétés antibiotiques.

L'invention a pour objet les composés de formule (I) : 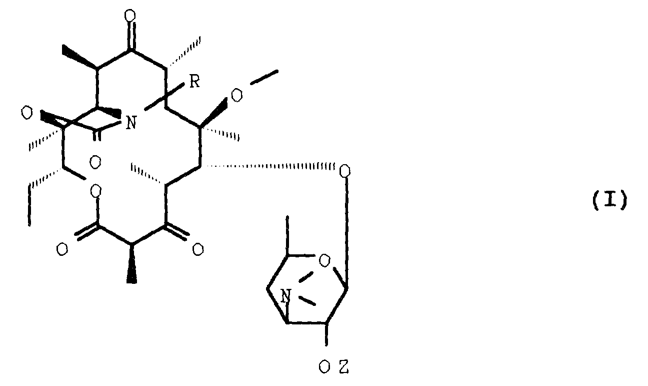 dans lesquels,
. ou bien R représente un radical (CH₂)₄ Ar₁, Ar₁ représentant :
   - soit un radical aryle carbocyclique renfermant jusqu'à 18 atomes de carbone, substitué par un ou plusieurs radicaux choisis dans le groupe constitué par les atomes d'halogènes, les radicaux alkyle, linéaire, ramifié ou cyclique , alkényle et alkynyle, linéaire ou ramifié, O-alkyle, O-alkényle et O-alkynyle, S-alkyle, S-alkényle ou S-alkynyle et N-alkyle, N-alkényle et N-alkynyle, renfermant jusqu'à 12 atomes, le radical 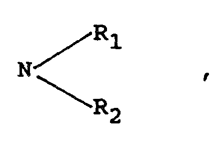 R₁ et R₂ identiques ou différents, représentant un atome d'hydrogène ou un radical alkyle renfermant jusqu'à 12 atomes de carbone, les radicaux aryle, O-aryle ou S-aryle carbocycliques ou aryle, éventuellement substitués par un ou plusieurs des substituants mentionnés ci-dessus,
   - soit un radical aryle hétérocyclique comportant un ou plusieurs hétéroatomes, éventuellement substitué par un ou plusieurs des substituants mentionnés ci-dessus,
. ou bien R représente un radical X Ar₂ dans lequel X représente un radical alkyle renfermant jusqu'à 6 atomes de carbone, interrompu par un groupement 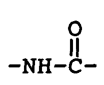 et Ar₂ représente un radical aryle ou hétéroaryle défini ci-dessus non substitué ou substitué par un ou plusieurs des substituants possibles de Ar₁ et Z représente un atome d'hydrogène ou un radical acyle renfermant jusqu'à 18 atomes de carbone ainsi que les sels d'addition avec les acides des composés de formule (I).

Comme exemple de sels d'addition des présents dérivés avec les acides minéraux ou organiques, on peut citer les sels formés avec les acides acétique, propionique, trifluoroacétique, maléique, tartrique, méthanesulfonique, benzènesulfonique, p-toluènesulfonique, chlorhydrique, bromhydrique, iodhydrique, sulfurique, phosphorique et spécialement les acides stéarique, éthylsuccinique ou laurylsulfurique.

Dans la définition des substituants :
- le radical aryle carbocyclique est de préférence le radical phényle ou naphtyle.
   Par radical aryle hétérocyclique, on entend soit un radical hétéroaryle monocyclique à 5 ou 6 chainons comportant un ou plusieurs hétéroatomes , soit un système polycyclique condensé, chaque cycle comprenant 5 ou 6 chainons et le cas échéant un ou plusieurs hétéroatomes.
- le radical aryle hétérocyclique comporte un ou plusieurs hétéroatomes choisis de préférence parmi l'oxygène, le soufre et l'azote.
- le radical hétéroaryle monocyclique à 5 chaînons est de préférence le radical thiényle, furyle, pyrrolyle, thiazolyle, oxazolyle, imidazolyle, thiadiazolyle, pyrazolyle ou isoxazolyle,
- le radical hétéroaryle monocyclique à 6 chaînons est de préférence un radical pyridyle, pyrimidinyle, pyridazinyle ou pyrazinyle,
- le radical hétéroaryle polycyclique condensé peut être par exemple le radical indolyle, benzofuryle, benzothiényle ou quinoléinyle, ou le reste d'une base purine telle que l'adenine,
- le radical alkyle, alkényle ou alkynyle est de préférence un radical méthyle, éthyle, propyle, isopropyle, n-butyle, isobutyle, terbutyle, décyle ou dodécyle, vinyle, allyle, éthynyle, propynyle, propargyle, cyclobutyle, cyclopentyle ou cyclohexyle,
- l'halogène est de préférence le fluor ou le chlore, ou le brome,
- le radical alkyle substitué par un atome d'halogène est de préférence un radical CHCl₂, CHBr₂, CHF₂, CCl₃, CBr₃, CF₃, CH₂CF₃, CH₂CH₂CCl₃, CH₂CH₂CF₃,
- le reste d'acide carboxylique est de préférence le reste acétyle, propionyle, butyryle, isobutyryle, n-valéryle, isovaléryle, tert-valéryle et pivalyle.

Parmi les composés préférés de l'invention, on peut citer :
a) les composés de formule (I) dans lesquels Z représente un atome d'hydrogène,
b) les composés de formule (I) dans lesquels R représente le radical (CH₂)₄ Ar₁, Ar₁ étant défini comme précédemment,
c) les composés de formule (I) dans lesquels Ar₁ représente un radical phényle substitué par l'un des radicaux énumérés précédemment, par exemple ceux dans lesquels Ar₁ représente un radical phényle substitué par un ou plusieurs atomes d'halogène, notamment un ou plusieurs atomes de chlore, et tout particulièrement les composés de formule (I) dans lesquels Ar₁ représente un radical 4-chloro phényle, ou encore ceux dans lesquels Ar₁ représente un radical phényle substitué par un ou plusieurs radicaux O-alkyle renfermant jusqu'à 4 atomes de carbone, notamment ceux dans lesquels Ar₁ représente un radical phényle substitué par un ou plusieurs radicaux méthoxy et tout particulièrement les composés de formule (I) dans lesquels Ar₁ représente un radical 4-méthoxyphényle,
d) les composés de formule (I) dans lesquels Ar₁ représente un radical aryle hétérocyclique à 5 chaînons, éventuellement substitué, par exemple un radical thiényle éventuellement substitué et plus spécialement le radical thiényle non substitué ou encore un radical imidazolyle éventuellement substitué et plus spécialement le radical imidazolyle non substitué.

Parmi les composés préférés de l'invention, on peut citer les composés de formule (I) dans lesquels Ar₁ représentent un radical biphényle éventuellement substitué. Par biphényle on entend un radical :

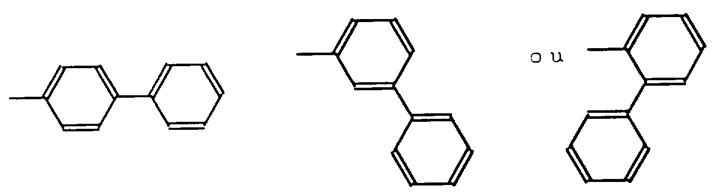

L'invention a tout spécialement pour objet les composés de formule (I) dans lesquels Ar₁ représente un radical : 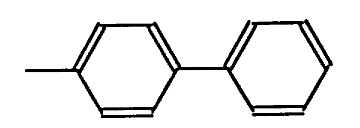 éventuellement substitué.

Parmi les composés de l'invention, on peut citer également les composés de formule (I) dans lesquels R représente un radical X₁Ar₂ dans laquelle X₁ représente un radical alkyle renfermant jusqu'à 6 atomes de carbone interrompu par un radical 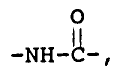 et Ar₂ conserve sa signification précédente.

Parmi ces composés, on peut citer tout spécialement les composés dans lesquels R représente un radical :

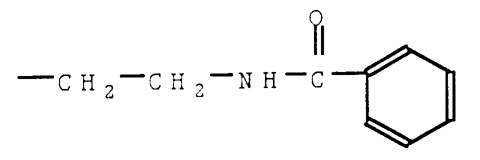

le radical phényle pouvant être éventuellement substitué par un ou plusieurs des substituants indiqués ci-dessus.

L'invention a tout spécialement pour objet les composés de formule (I), dont la préparation est donnée ci-après et tout particulièrement les composés des exemples 1, 2, 3, 4, 11 et 15.

Comme composés particulièrement intéressants, on peut également citer le produit de l'exemple 28 ou encore les produits des exemples 38 et 39.

Les produits de formule générale (I) possèdent une très bonne activité antibiotique sur les bactéries gram ^{⊕} telles que les staphylocoques, les streptocoques, les pneumocoques.

Les composés de l'invention peuvent donc être utilisés comme médicaments dans le traitement des infections à germes sensibles et notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, bronchopneumonies, suppuration pulmonaires, les streptococcies telles que les angines aiguës, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Rickettsies, Mycoplasma pneumoniae, Chlamydia, Legionella, Ureaplasma, Toxoplasma ou à des germes du genre Mycobactérium, Listeria, Meningocoques et Campylobacter.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, les produits préférés de formule (I) définis précédemment à savoir les produits des exemples 1, 2, 3, 4, 11 et 15, ainsi que leurs sels pharmaceutiquement acceptables.

On peut également citer à titre de médicaments, le produit de l'exemple 28 ainsi que les produits des exemples 38 et 39 et leurs sels pharmaceutiquement acceptables.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés, simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour le produit de l'exemple 2 ou de l'exemple 11.

L'invention a également pour objet un procédé de préparation des composés de formule (I), caractérisé en ce que l'on soumet un composé de formule (II) : 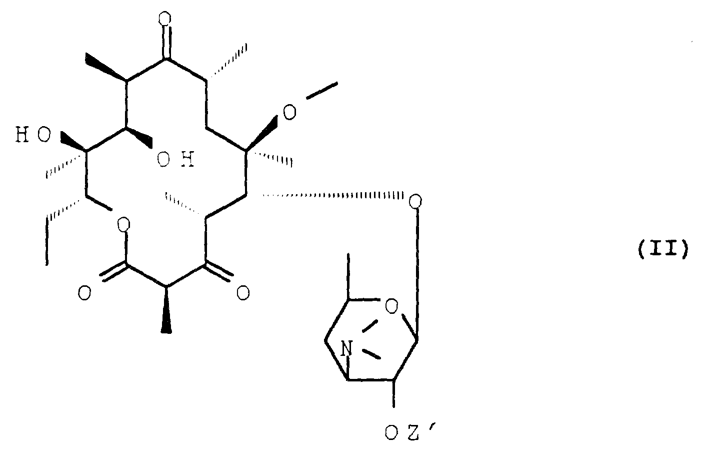 dans laquelle Z' représente le reste d'un acide carboxylique renfermant jusqu'à 18 atomes de carbone, à l'action d'un agent capable d'activer sélectivement l'hydroxyle en 11 pour obtenir le composé de formule (III) : 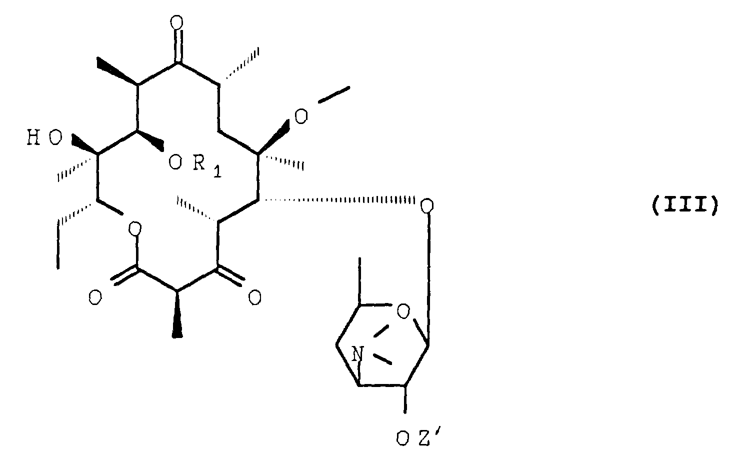 dans laquelle R₁ représente le reste d'un groupement facilement clivable, que l'on soumet à l'action d'une base, pour obtenir le composé de formule (IV) : 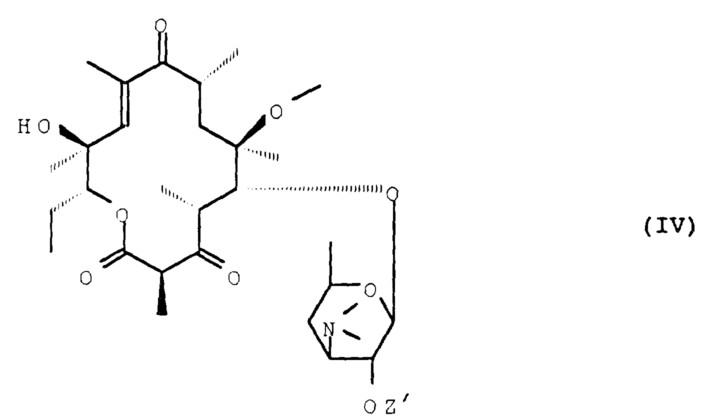 puis soumet le composé de formule (IV) :
- soit à l'action d'un composé de formule (V) :

   R-N=C=O (V)

   dans laquelle R a la signification indiquée précédemment, pour obtenir le composé de formule (VI) : 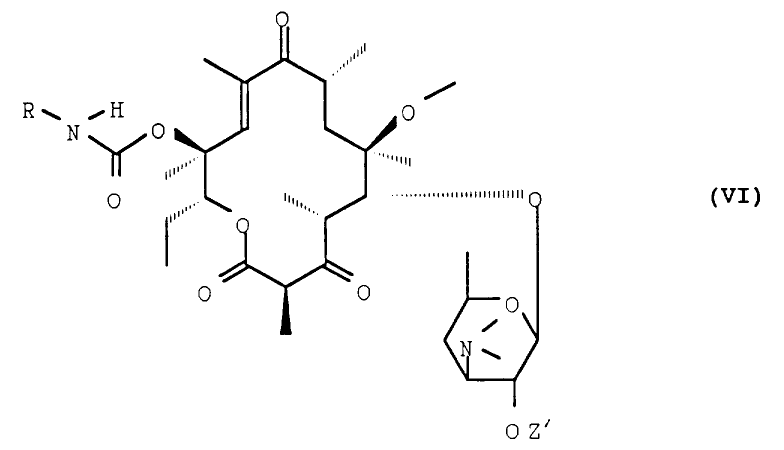 qui se cyclise soit spontanément par chauffage, soit que l'on soumet à l'action d'un agent de cyclisation pour obtenir le composé de formule (IA) : 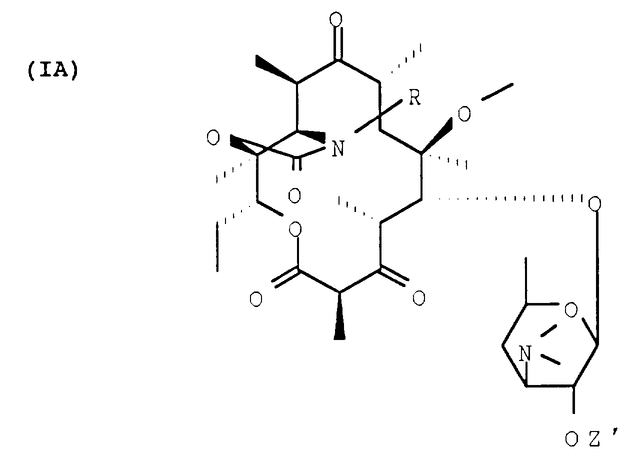 correspondant au produit de formule (I) dans laquelle Z ne représente pas un atome d'hydrogène,
- soit à l'action du carbonyldiimidazole pour obtenir le composé de formule (VII) : 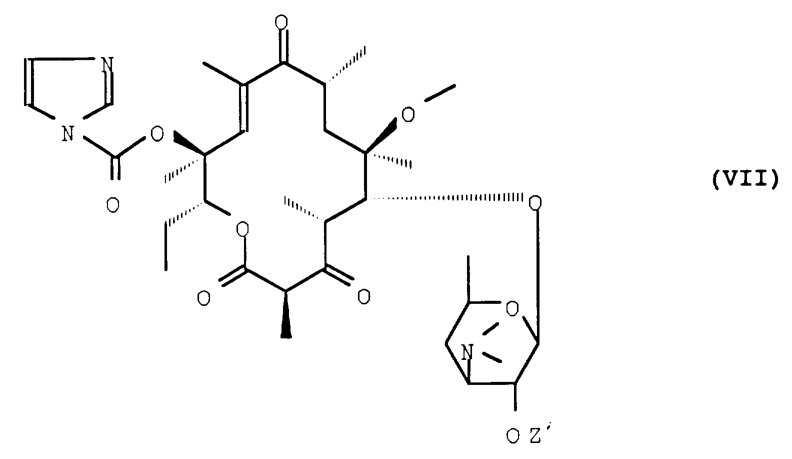 puis à l'action d'un composé de formule (VIII) :

   RNH₂ (VIII)

   dans laquelle R a la signification indiquée ci-dessus, pour obtenir le composé de formule (VI) défini précédemment qui se cyclise soit spontanément par chauffage, soit que l'on soumet à l'action d'un agent de cyclisation pour obtenir le composé de formule (IA) correspondant, puis soumet le cas échéant, le composé de formule (IA) à l'action d'un agent de libération de la fonction hydroxyle en 2' et/ou le cas échéant, à l'action d'un acide pour en former le sel.

Dans un mode de réalisation préféré du procédé de l'invention :
- l'agent capable d'activer sélectivement l'hydroxyle en 11 est un anhydride sulfonique tel que l'anhydride méthanesulfonique, paratoluène sulfonique ou trifluorométhane sulfonique,
- la base utilisée pour créer une double liaison 10(11) est un diazabicycloundécène, par exemple le DBU (ou 1,8 diazabicyclo[5-4-0]undec-7-ène), ou le diazabicyclononène, ou la 2,6-lutidine, ou la 2,4,6-collidine ou la tétraméthylguanidine,
- la réaction entre le composé de formule (IV) et le composé de formule (V) a lieu en présence d'une base, comme la pyridine, la triéthylamine, la morpholine, la N-méthyl morpholine, la cyclisation du composé de formule (VI) intervenant soit spontanement, soit par chauffage à une température comprise entre 50° et 100° C.
- la réaction du composé de formule (IV) avec le carbonyldiimidazole a lieu en présence d'une base comme l'hydrure de sodium, ou la triéthylamine, ou un carbonate ou un carbonate acide de sodium ou de potassium, ou en l'absence de base dans un solvant tel que le chlorure de méthylène, le tétrahydrofuranne, ou le dimethylformamide,
- la réaction du composé de formule (VII) avec le composé RNH₂ a lieu au sein d'un solvant tel que par exemple l'acétonitrile, le diméthylformamide ou encore le tétrahydrofuranne, le diméthoxy éthane ou le diméthylsulfoxyde, la cyclisation du composé de formule (VI) se produisant en général au cours de la réaction ou intervenant par action, sur le composé de formule (VI) isolé, d'une base telle que le terbutylate de potassium, au sein d'un solvant tel que le tetrahydrofuranne,
- l'hydrolyse de la fonction ester en 2' est réalisée à l'aide du méthanol ou de l'acide chlorhydrique aqueux,
- la salification est réalisée au moyen d'acides selon les procédés classiques.

Les composés de formule (II) utilisés comme produits de départ sont des produits connus d'une façon générale et peuvent être préparés comme il est indiqué dans la demande de brevet européen 0 487 411.

Les composés de formule RN=C=O et RNH₂ sont des produits connus d'une façon générale.

Les composés de formule RNH₂ peuvent par exemple être préparés selon les procédés décrits dans J. Med. Chem (1982) vol. 25 p. 947 et suivantes, Tetrahedron Letters vol. 32, n° 14, p. 1699-1702, (1991) ; J. Org. Chem. 54 (18) 4298, 301 (1989) ; J. Org. Chem. 28 (101) 2589 91 (1963 ou le brevet allemand 3 406 416. ; J. Org. Chem. 6-895-901 (1941) ou Synth. Commun 17 (14) 1741-8 (1987.

Certains produits de formule (VIII) sont des produits nouveaux et sont en eux-mêmes un objet de la présente invention, leur préparation est donnée ci-après dans la partie expérimentale.

Ainsi les produits suivants sont des produits nouveaux et sont en eux-mêmes un objet de la présente invention :
- la 4-(2-thiényl) butylamine,
- la 4-(1,1'biphényl) butylamine,
- la 4-(4-méthylphényl) butylamine,
- la 4-(2,4-diméthylphényl) butylamine,
- la 4-(2,4,6-triméthylphényl) butylamine,
- la 4-(2-méthoxyphényl) butylamine.

L'invention s'étend naturellement aux produits obtenus lors de la mise en oeuvre du procédé de l'invention, à savoir les produits de formules (III), (IV), (VI) et (VII) et tout particulièrement au composé de formule (IV) dont la préparation est donnée ci-après dans la partie expérimentale et au composé 2'-OH correspondant.

Les exemples suivants illustrent l'invention.

### EXEMPLE 1 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-chlorophényl) butyl) imino)) érythromycine

### STADE A : 2'-acétate de 3-de((2,6-didéoxy 3-C-méthyl 3-O-méthyl alpha-L-ribohexopyranosyl) oxy) 6-O-méthyl 11-O-(méthylsulfonyl) 3-oxo érythromycine

On introduit sous agitation et sous atmosphère d'azote 17 g de 2'-acétate de 3-de((2,6-didéoxy 3-C-méthyl 3-O-méthyl alpha-L-ribohexopyranosyl) oxy) 6-O-méthyl 3-oxo érythromycine dans 100 ml de pyridine. On refroidit le mélange obtenu jusqu'à 10°C. On ajoute 11,9 g d'anhydride méthane sulfonique. On laisse revenir à la température ambiante. On maintient l'agitation pendant 5 heures. On filtre le précipité obtenu. On concentre, reprend à l'eau et extrait à l'acétate d'éthyle. On lave à l'eau les phases organiques, les sèche, filtre et concentre. On obtient ainsi 20,9 g de produit recherché brut que l'on purifie par salification à l'aide d'acide oxalique puis libération de la base avec de l'ammoniaque.

On obtient ainsi 15,16 g du produit recherché (F = 210-212°C).

### STADE B : 2'-acétate de 11-déoxy 10,11-didéhydro 3-de(2,6-didéoxy 3-C-méthyl 3-O-méthyl alpha-L-ribohexopyranosyl) oxy) 6-O-méthyl 3-oxo érythromycine

On introduit sous agitation 8,26 g de produit préparé au stade A dans 35 ml d'acétone. On ajoute ensuite goutte à goutte 2,19 ml de DBU. On maintient l'agitation à la température ambiante pendant 20 heures. On reprend le mélange réactionnel avec du chlorure de méthylène. On lave à l'eau les phases organiques, les sèche sur sulfate de sodium, filtre et concentre. On obtient 10 g de produit que l'on reprend dans l'éther. On essore et lave avec de l'éther éthylique. On obtient ainsi 6,33 g de produit recherché (F = 230-232°C).

### STADE C : 2'-acétate de 11-déoxy 10,11-didéhydro 3-de(2,6-didéoxy 3-C-méthyl 3-O-méthyl alpha-L-ribohexopyranosyl) oxy) 12-O-((1H-imidazol-1-yl) carbonyl) 6-O-méthyl 3-oxo érythromycine

On introduit 96 mg d'hydrure de sodium à 50 % dans l'huile dans 15 ml de tetrahydrofuranne. On refroidit la suspension obtenue à 0°C et introduit goutte à goutte une solution de 611 mg de produit préparé au stade précédent dans 17 ml de tetrahydrofuranne. On introduit à 0°C une solution de 486 mg de carbonyldiimidazole dans 15 ml de tetrahydrofuranne. On maintient l'agitation pendant 4 h 30. On laisse le mélange réactionnel revenir à la température ambiante, filtre et concentre. On reprend avec de l'acétate d'éthyle, lave avec une solution de dihydrogénophosphate de sodium, extrait avec de l'acétate d'éthyle, sèche, filtre et concentre. On obtient 852 mg de produit recherché, que l'on utilise tel quel dans le stade suivant.

### STADE D : 2'-acétate de 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-chlorophényl) butyl) imino)) érythromycine

On ajoute aux 852 mg de produit préparé au stade précédent une solution renfermant 1,1 g de 4-(4-chlorophényl) butylamine (préparée comme indiqué dans J. Med. Chem. 1982 Vol. 25 n° 951), 3 ml d'acétonitrile et 0,3 ml d'eau déminéralisée.

On agite 4 heures à 55°C. On verse le milieu réactionnel sur une solution de dihydrogénophosphate de sodium, extrait au chlorure de méthylène, lave à l'eau, sèche, filtre et concentre. On obtient 1,4 g d'huile que l'on chromatographie sur silice en éluant avec le mélange chlorure de méthylèneisopropanol (95-5). On rassemble les fractions homogènes en CCM, filtre et concentre. On obtient 0,44 g d'une huile que l'on empâte dans l'éther isopropylique, essore et sèche à 70°C. On obtient ainsi 0,262 g de produit recherché (F = 179-181°C).

### STADE E : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-chlorophényl) butyl) imino)) érythromycine

On maintient sous agitation un mélange de 0,23 g de produit préparé au stade précédent et 6 ml de méthanol. On maintient l'agitation à la température ambiante pendant 15 heures. On évapore le méthanol, chromatographie sur silice en éluant avec le mélange acétate d'éthyle-méthanol-ammoniaque (9-1-0,01). On rassemble les phases homogènes en CCM, filtre et concentre. On obtient 0,14 g d'huile que l'on empâte dans l'éther isopropylique, essore et sèche à 80°C sous pression réduite. On obtient 0,094 g de produit (F = 194-196°C).
α_{D} : +23° CHCl₃ (1 %).

### EXEMPLE 2 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-méthoxyphényl) butyl) imino)) érythromycine

### STADE A : 2'-acétate de 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-méthoxyphényl) butyl) imino)) érythromycine

En opérant comme à l'exemple 1, stade D, à partir de 0,8 g de 2'-acétate de 11-déoxy 10,11-didéhydro 3-de((2,6-didéoxy 3-C-méthyl 3-O-méthyl alpha-L-ribohexopyranosyl) oxy) 12-O-((1H-imidazol-1-yl) carbonyl) 6-O-méthyl 3-oxo érythromycine, 3 ml d'acétonitrile et 1,0 g de 4-(4-méthoxyphényl) butylamine préparée selon Tetrahedron Letters 32, 1699-1702, (1991) on obtient 0,8 g du produit recherché sous la forme d'un mélange de produit acétylé et désacétylé en 2'. Chromatographie sur silice chlorure de méthylène-méthanol (9-1). rf = 0,47.

### STADE B : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-méthoxyphényl) butyl) imino)) érythromycine

En opérant comme à l'exemple 1, stade E, à partir de 0,8 g du produit brut obtenu au stade précédent, on obtient 0,237 g du produit recherché (F = 193-195°C).
α_{D} : +22°3 (C = 1 % CHCl₃).

### EXEMPLE 3 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(2-thiényl) butyl) imino)) érythromycine

### STADE A : 2'-acétate de 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(2-thiényl) butyl) imino)) érythromycine

En opérant comme à l'exemple 1, stade D, à partir de 0,85 g de 2'-acétate de 11-déoxy 10,11-didéhydro 3-de((2,6-didéoxy 3-C-méthyl 3-O-méthyl alpha-L-ribohexopyranosyl)oxy 12-O-((1H-imidazol-1-yl) carbonyl) 6-O-méthyl 3-oxo érythromycine, 3 ml d'acétonitrile, 0,3 ml d'eau et 0,822 g de 4-(2-thiényl) butylamine (dont la préparation est donnée ci-après) on obtient 0,212 g de produit recherché (F = 218-220°C).

### STADE B : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(2-thiényl) butyl) imino)) érythromycine

En opérant comme à l'exemple 1, stade E, à partir de 0,182 g de produit préparé au stade précédent et 6 ml de méthanol, on obtient 0,085 g de produit recherché (F = 188-190°C).
α_{D}: + 24° (C = 1 % CDCl₃).

### EXEMPLE 4 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(1,1'-biphényl) 4-yl) butyl) imino)) érythromycine

### STADE A : 2'-acétate de 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(1,1'-biphenyl)4-yl) butyl) imino)) érythromycine

On chauffe à 55°C pendant 5 heures, un mélange renfermant 3,5 ml d'acétonitrile, 0,7 g de 2'-acétate de 11-déoxy 10,11-didéhydro 3-de((2,6-didéoxy 3-C-méthyl 3-O-méthyl alpha-L-ribohexopyranosyl) oxy) 12-O-((1H-imidazol-1-yl) carbonyl) 6-O-méthyl 3-oxo érythromycine, 0,3 ml d'eau, 1,1 g de biphénylbutylamine (préparation donnée ci-après).

On verse le milieu réactionnel sur une solution aqueuse saturée de dihydrogénophosphate de sodium, extrait à l'acétate d'éthyle et filtre. On laisse décanter la phase aqueuse, extrait à l'acétate d'éthyle et lave à l'eau. On sèche, filtre et concentre. On obtient 1,1 g de produit attendu.

### STADE B : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(1,1'-biphenyl)4-yl) butyl) imino)) érythromycine

on verse les 1,1g de produit obtenu au stade A dans 12 ml de méthanol. On agite la solution obtenue pendant 15 heures à la température ambiante. On concentre, dilue avec 3 ml de chlorure de méthylène, sèche, filtre, concentre. On obtient un produit que l'on recristallise dans un mélange éther isopropylique, éther éthylique (9-1). On essore, sèche à 80°C et obtient 0,148 g de produit fondant à 166∼168°.
RMN - CDCl₃
1,34 (s) et 1,47 (s) - 6 et 12 CH₃ ; 2,68 (m) - CH₂-Φ ; 3,05 à 3,25 - H₁₀, H₄ et H₂' ; 3,60(s) -H₁₁ ; 3,67(m) 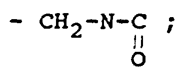 ∼ 7,26 - ∼ 7,49 - phényl interne ; 7,31 H en para,
∼ 7,42 H en méta, ∼ 7,58 H en ortho - phényl externe.

### Microanalyse :

| % théoriques | | % trouvés | |
|---|---|---|---|
| C % | 68,75 | C % | 68,6 |
| H % | 8,35 | H % | 8,5 |
| N % | 3,41 | N % | 3,3 |

En opérant comme précédemment, à partir du 2'-acétate de 11-déoxy 10,11-didéhydro 3-de((2,6-didéoxy 3-C-méthyl 3-O-méthyl alpha-L-ribohexopyranosyl) oxy) 12-O-((1H-imidazol-1-yl) carbonyl) 6-O-méthyl 3-oxo érythromycine, et des amines appropriées, on a obtenu les produits suivants :

### EXEMPLE 5 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(2-méthoxyphényl) butyl) imino)) érythromycine

F = 190 ∼192°C.
α_{D} : + 24° (C = 1 % CHCl₃).

### EXEMPLE 6 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((2-(phénylméthylthio) éthyl) imino)) érythromycine

F = 190 ∼ 192°C.
α_{D} : - 11,5° (C = 1 % CHCl₃).

### EXEMPLE 7 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-nitrophényl) butyl) imino)) érythromycine

F = 200 ∼ 202°C.
α_{D} : + 15,5° (C = 1 % CHCl₃).

### EXEMPLE 8 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(2,4-diméthylphényl) butyl) imino)) érythromycine

F = 183 ∼ 185°C.
α_{D} : + 21° (C = 1 % CHCl₃).

### EXEMPLE 9 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-méthylphényl) butyl) imino)) érythromycine

F = 200 ∼ 202°C.
α_{D} : + 23° (C = 1 % CHCl₃).

### EXEMPLE 10 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(2,4,6-triméthylphényl) butyl) imino)) érythromycine

F = 188 - 190°C.
α_{D} : + 24° (C = 1 % CHCl₃).

### EXEMPLE 11 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-((1H)-imidazol-1-yl) butyl) imino)) érythromycine

F = 212 - 214°C.
α_{D} : + 26,2° (C = 0,85 % CHCl₃) .

### EXEMPLE 12 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(3-méthoxyphényl) butyl) imino)) érythromycine

F = 196 - 198°C.
α_{D} : + 18,8° (C = 1 % CHCl₃).

### EXEMPLE 13 : 11,12-didéoxy 2-de((2,6-didéoxy 3-C-méthyl 3-O-méthyl alpha-L-ribohexopyranosyl) oxy) 6-O-méthyl 3-oxo 12,11-(oxycarbonyl ((4-(phénylamino) 4-oxobutyl) imino)) érythromycine

F = 190-192°C.
α_{D} = +8° (C = 1 % CHCl₃).

### EXEMPLE 14 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((3-(phénylthio) propyl) imino)) érythromycine

F = 204 - 206°C.
α_{D} : + 19° (C = 0,9 % CHCl₃).

### EXEMPLE 15 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((2-(((2-((phénylcarbonyl) amino) éthyl) imino)) érythromycine

F = 240°C
α_{D} : - 2° (C = 1 % CHCl₃).

### EXEMPLE 16 : 11,12-didéoxy 3-de((2,6-didéoxy 3-C-méthyl 3-O- methyl alpha-L-ribohexopyranosyl) oxy) 6-O-méthyl 3-oxo 12,11-(oxycarbonyl ((3-phénoxypropyl) imino)) érythromycine

F = 222 ∼ 225°C.
α_{D} : + 20° (C = 0,9 % CHCl₃).

### EXEMPLE 17 : 11,12-didéoxy 3-de((2,6-didéoxy 3-C-méthyl 3-O-méthyl alpha-L-ribohexopyranosyl) oxy) 6-O-méthyl 3-oxo 12,11- ((2-(phénylméthoxy) éthyl) imino)) érythromycine

F = 207°C.

### EXEMPLE 18 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((2-(4-méthoxyphényl) éthyl) imino)) érythromycine

F = 218 ∼ 220°C.
α_{D} : + 15,5° (C = 1 % CHCl₃).

### EXEMPLE 19 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(1H-indol-4-yl) butyl) imino)) érythromycine

F = 208 ∼ 212°C.
α_{D} : + 22° (C = 1 % CHCl₃).

### EXEMPLE 20 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(3-aminophényl) butyl) imino)) érythromycine

F = 200 ∼ 202°C et 210°C.
α_{D} : + 23° (C = 1 % CHCl₃).

### EXEMPLE 21 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(2-chlorophényl) butyl) imino)) érythromycine

F = 193 ∼ 195°C.
α_{D} : +23° (C = 1 % CHCl₃).

### EXEMPLE 22: 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(3-chlorophényl) butyl) imino)) érythromycine

F = 191 ∼ 193°C.
α_{D} : + 22° (C = 1 % CHCl₃).

### EXEMPLE 23 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-hydroxyphényl) butyl) imino)) érythromycine

F = 220 ∼ 222°C.

### EXEMPLE 24 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-(1-oxobutyl) phényl) butyl) imino)) érythromycine

F = 134 ∼ 136°C.

### EXEMPLE 25 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-(1-oxoéthyl) phényl) butyl) imino)) érythromycine

F = 170 ∼ 172°C.

### EXEMPLE 26 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((3-(phénylsulfonyl) propyl) imino)) érythromycine

F = 202 ∼ 204°C.
α_{D} : + 21° (C = 1 % CHCl₃).

### EXEMPLE 27 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-butylphényl) butyl) imino)) érythromycine

F = 134 ∼ 135°C.
α_{D} : + 19,5° (C = 1 % CHCl₃).

### EXEMPLE 28 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-quinoléiny) butyl) imino)) érythromycine

F = 170 ∼ 172°C.

### EXEMPLE 29 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-((2,2'-bithiophen) 5-yl) butyl) imino)) érythromycine

F = 147 ∼ 149°C.

### EXEMPLE 30 : 11,12-didéoxy 3-de((2,6-didéoxy 3-C-méthyl 3-O-méthyl

### alpha-L-ribohexopyranosyl) oxy) 6-O-méthyl 3-oxo 12,11-(oxycarbonyl ((3-oxo 3-((2-thiazolyl) amino) propyl) imino)) érythromycine

### EXEMPLE 31 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-éthylphényl) butyl) imino)) érythromycine

F = 191 ∼ 193°C.
α_{D} : + 20° (C = 1 % CHCl₃).

### EXEMPLE 32 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-(4-chlorobenzoyl) 3-méthylphényl) butyl) imino)) érythromycine

F = 143 - 145°C.
α_{D} : + 8° (C = 1 % CHCl₃).

### EXEMPLE 33 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl (4-(9H-fluoren-2-yl)butyl) imino)) érythromycine

F = 215 ∼ 217°C.
α_{D} : + 20° (C = 1% CHCl₃).

### EXEMPLE 34 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl (4-(6-amino-9H-purin-9-yl)butyl) imino)) érythromycine

α_{D} : + 13° (C = 1% CHCl₃).

### EXEMPLE 35 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl (4-(4-phenoxyphenyl)butyl) imino)) érythromycine

F = 154 ∼ 156°C
α_{D} : + 16° (C = 1% CHCl₃).

### EXEMPLE 36 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl (4-(2-phenyl-1H-imidazol-1-yl)butyl) imino)) érythromycine

F= 132 - 134°C
α_{D} : + 13° (C = 1% CHCl₃).

### EXEMPLE 37 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl (4-(4-fluorophenyl)butyl) imino)) érythromycine

F = 180°C

### EXEMPLE 38 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl (4-(1H-benzimidazol-1-yl)butyl) imino)) érythromycine

F= 942 - 196°C
RF + 0,43 (CHCl₂-MeOH-NH₄OH 95-5-0,2)

### EXEMPLE 39 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl (4-(2-phényl-5-thiazolyl)butyl) imino)) érythromycine

F= 118 - 120°C
rf = 0,24 (AcOEt à 4% de TEA)

En opérant comme précédemment, on a préparé les composés de formule (I) suivant :

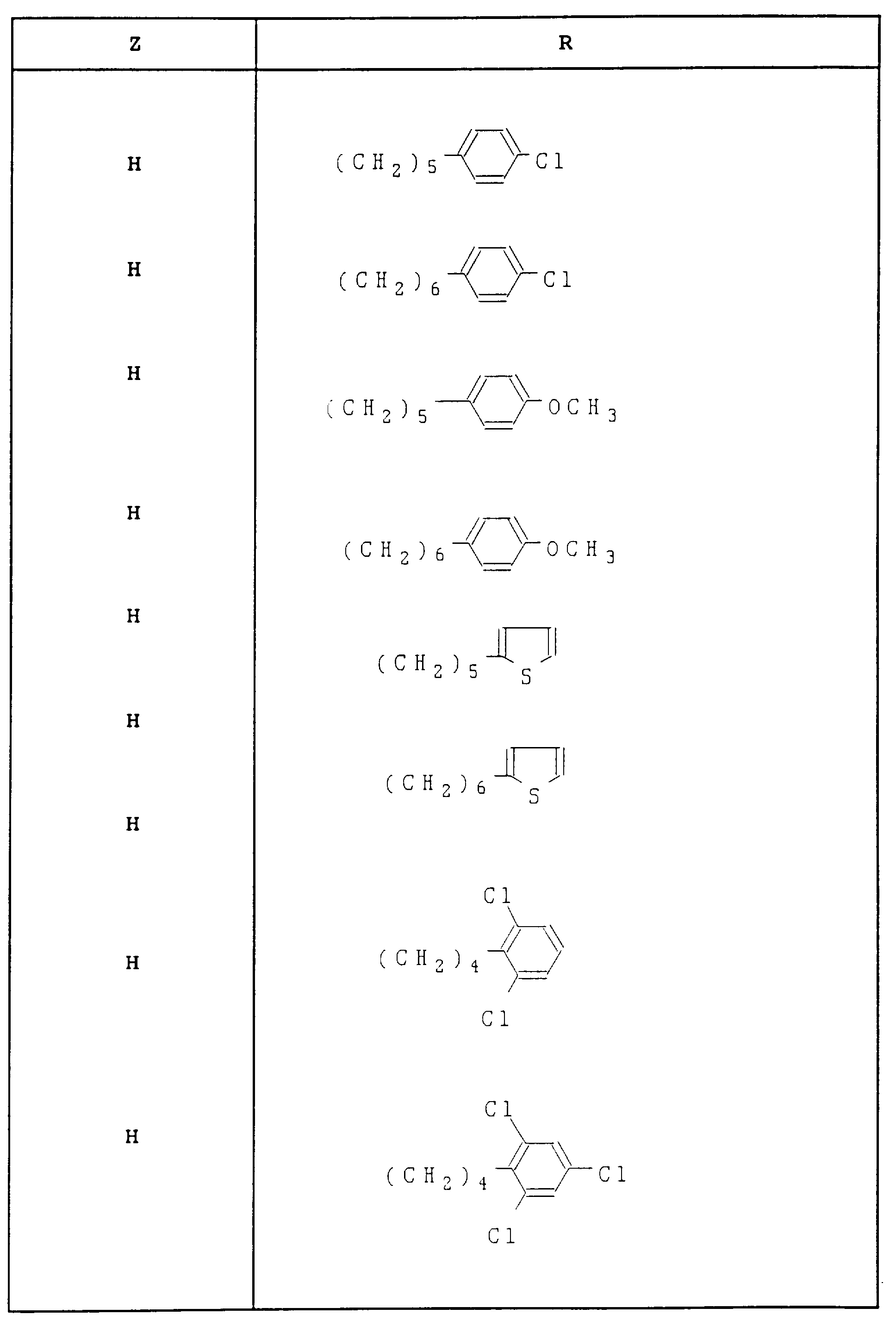

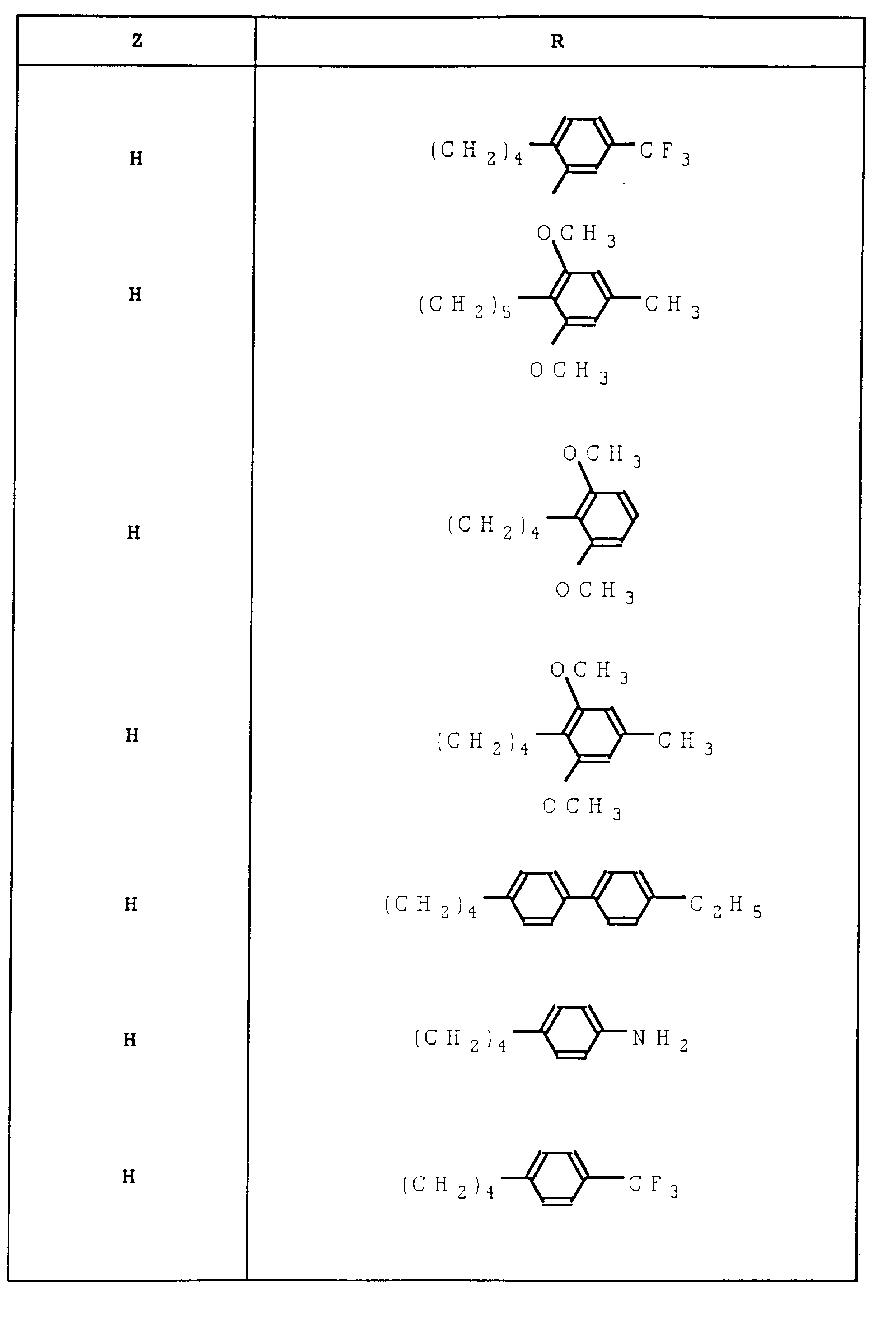

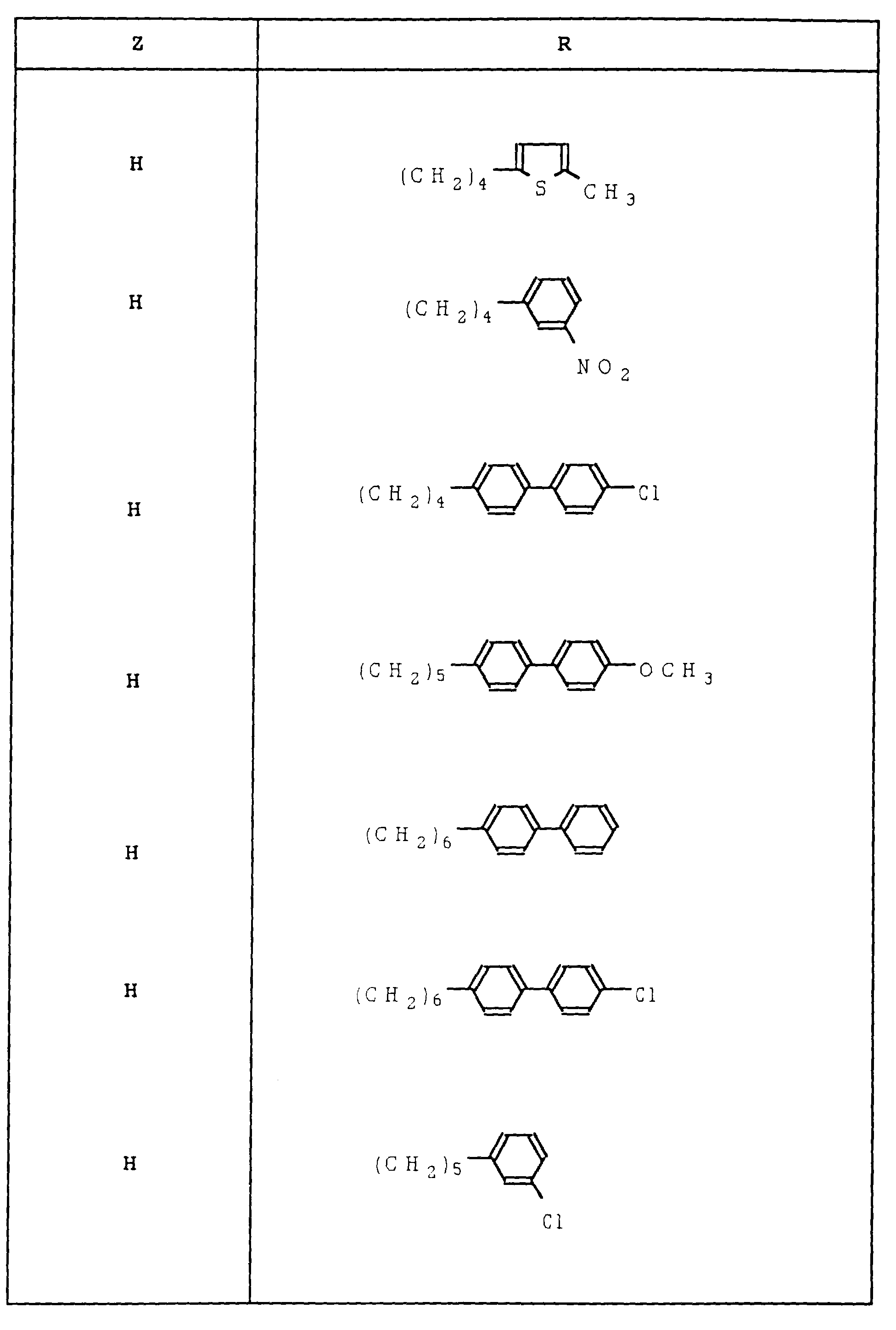

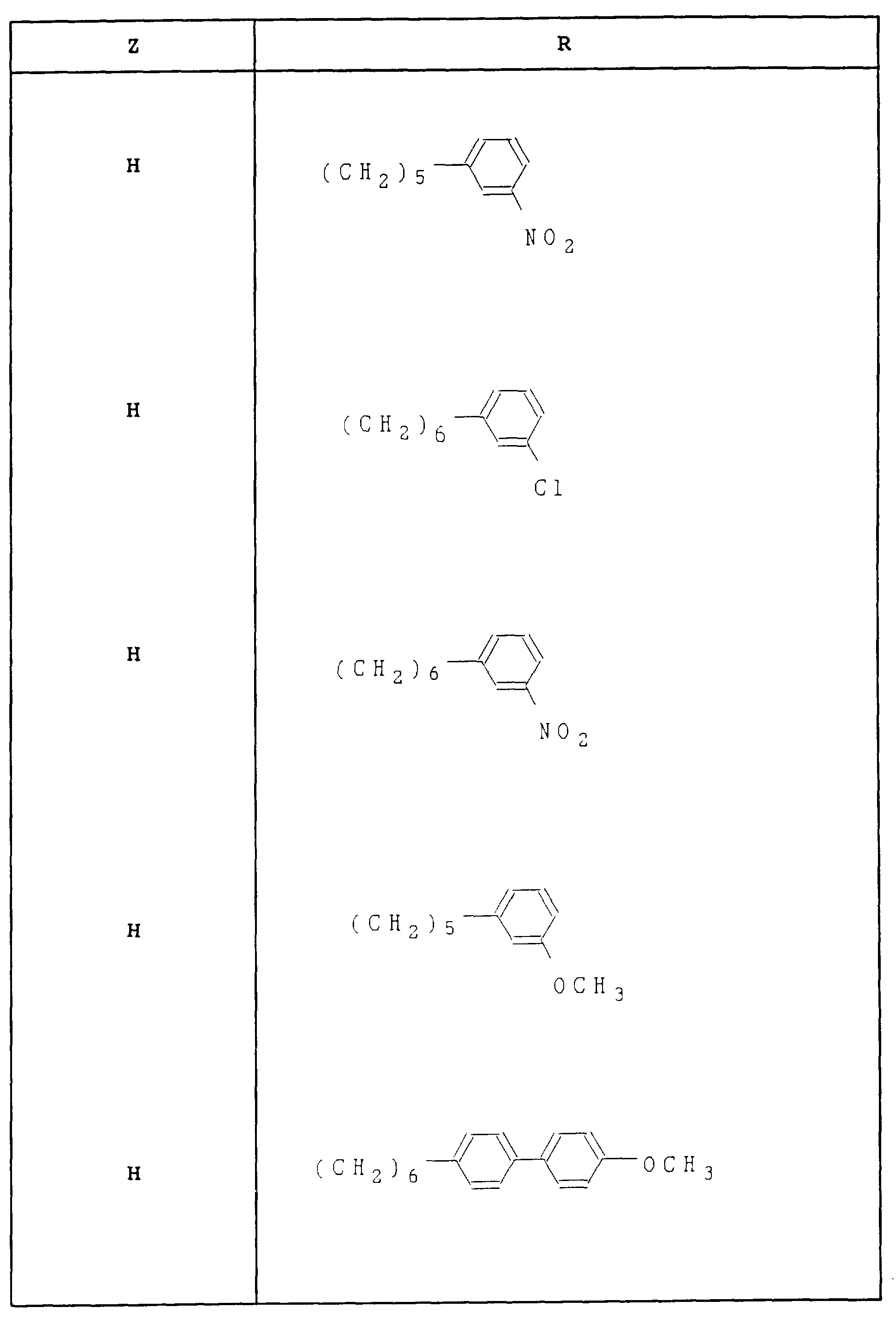

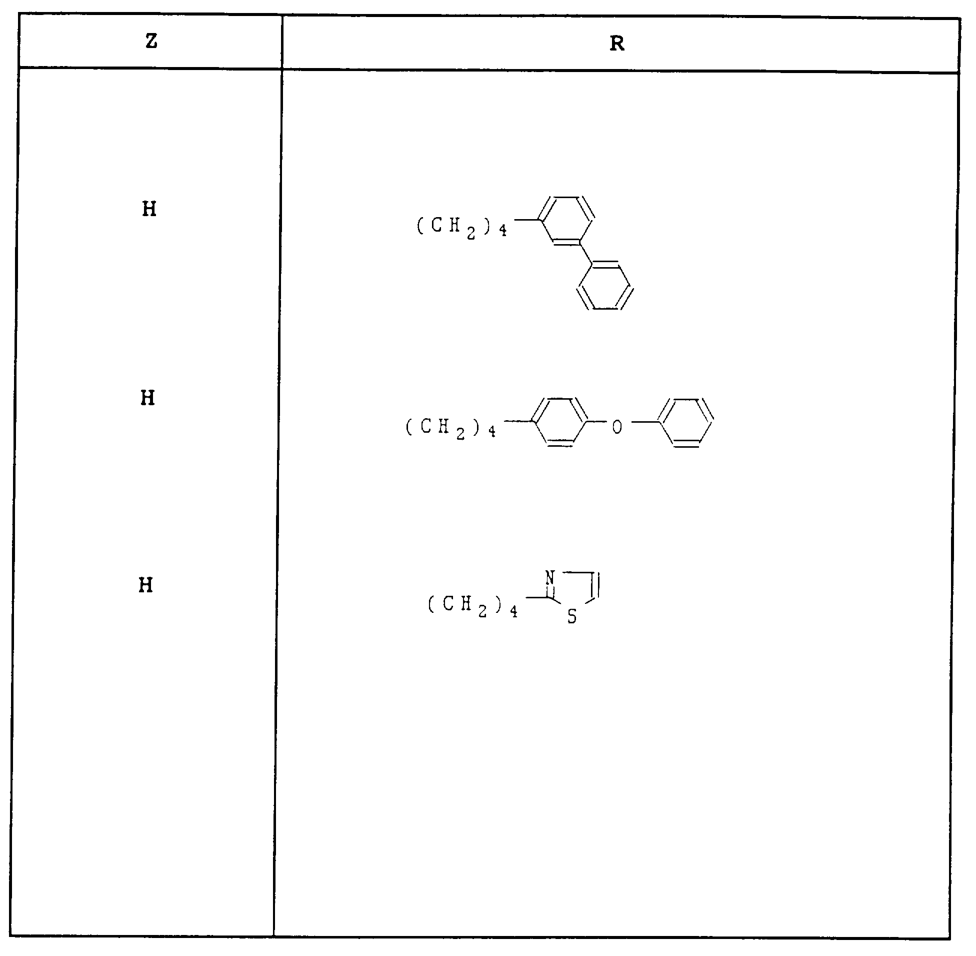

### PREPARATION 1 : 4-(2-thiényl) butylamine

### STADE A : 4-(2-thiényl) butylamide.

On agite 3 heures à 60°C un mélange de 40 ml de dichloréthane, 5,1 ml d'acide 4-(2-thiényl) butyrique, et 10,2 ml de chlorure de thionyle. On évapore le dichloroéthane, verse le produit obtenu sur de l'ammoniaque concentrée, refroidie à 0°C. On essore et sèche le produit obtenu. On obtient 4,29 g de produit que l'on chromatographie sur silice en éluant avec le mélange chlorure de méthylène-méthanol (92-8). On rassemble les fractions homogènes en CCM, concentre et filtre. On empâte le produit obtenu dans l'éther isopropylique, essore et sèche. On obtient 1,18 g de produit recherché (F = 84∼86°C).

### STADE B : 4-(2-thiényl) butylamine

On introduit goutte à goutte à 0°C, 1,1 g de 4-(2-thiényl) butylamide dans un mélange renfermant 30 ml de tétrahydrofuranne et 1,06 g d'hydrure de lithium aluminium. On laisse revenir à la température ambiante, agite à température ambiante pendant 4 h 30, puis 1 heure à 30°C, et 16 heures à température ambiante. On refroidit à 0°C, ajoute 3 ml d'un mélange eau-tetrahydrofuranne (2-1) puis 8 ml d'eau, puis 6 ml d'une solution saturée de sel double de tartrate de sodium et de potassium. On filtre et concentre. On reprend le produit obtenu à l'éther éthylique, lave au carbonate de sodium puis à l'eau et extrait les phases aqueuses à l'éther éthylique. On rassemble les phases organiques, les sèche sur sulfate de sodium, filtre et concentre. On obtient 0,91 g d'un produit que l'on dissout dans 12 ml d'un mélange acétate d'éthyle-éthanol (95-5). On refroidit à 0°C et ajoute une solution d'acide chlorhydrique gazeux dans l'acétate d'éthyle. Le chlorhydrate du produit attendu précipite. On essore, sèche et obtient 0,675 g de produit recherché sous forme de chlorhydrate
(F = 168∼170°C). La base correspondante est obtenue par traitement alcalin, extraction à l'acétate d'éthyle, séchage sur sulfate de sodium, filtration et concentration.

### PREPARATION 2 : 4-[(1,1'-biphényl)-4-yl] butylamine

### STADE A : N-[4-[(1,1'-biphenyl)-4-yl] 3-butenyl]phtalimide

On refroidit à -40°C, une suspension renfermant 150 ml de tétrahydrofuranne, 5,46 g de 4-phénylbenzaldéhyde et 15,9 g de bromure triphényl phosphonium du N-(3-bromopropyl) phtalimide. On introduit ensuite 3,37 g de terbutylate de potassium. On laisse la température remonter jusqu'à -15°C et maintient l'agitation à -15°C pendant 1 heure.

On verse sur de la glace, extrait à l'acétate d'éthyle, lave à l'eau, sèche les phases organiques sur Na₂SO₄, filtre et concentre. On obtient 19 g de produit que l'on dissout dans le chlorure de méthylène et chromatographie sur silice en éluant avec le mélange acétate d'éthyle-hexane (3-7). On concentre. On empâte dans l'hexane, on essore, on sèche sous pression réduite et isole 8,5 g de produit recherché fondant à 112 ∼ 114°C.

### Microanalyse :

| % calculé | | % trouvé | |
|---|---|---|---|
| C % | 81,56 | C % | 81,4 |
| H % | 5,42 | H % | 5,3 |
| N % | 3,96 | N % | 3,8 |

### STADE B : 4-[(1,1'-biphényl) 4-yl] 3-butenylamine

On porte au reflux un mélange renfermant 280 ml d'éthanol, 7,9 g de produit préparé au stade A, et 1,3 ml d'hydrate d'hydrazine. On laisse revenir à la température ambiante, filtre le précipité obtenu et le lave à l'éthanol. On concentre, verse sur une solution d'acide chlorhydrique 2N et extrait à l'acétate d'éthyle. On lave à l'eau les phases organiques, les sèche, filtre et concentre sous pression réduite. On obtient 2,89 g de produit recherché.
F = 188 ∼ 194°C.

### STADE C : 4-[(1,1'-biphényl) 4-yl] butylamine

On place dans un appareil à hydrogène, 17 ml de méthanol, 1,74 g de produit préparé au stade B et 0,17 g de catalyseur Pd 10 % sur charbon. On maintient l'hydrogénation pendant une nuit. On filtre, lave et concentre. On empâte à l'acétate d'éthyle le produit obtenu, glace, essore et sèche sous pression réduite à 80°C. On obtient 1,55 g de produit recherché fondant à 260°C.

### PREPARATION 3: 4-(2,4,6-triméthylphényl) butylamine

En opérant comme à la préparation 2, on a obtenu le produit recherché sous forme de chlorhydrate.
F = 218 ∼ 220°C.

### PREPARATION 4 : 4-(4-méthylphényl) butylamine

En opérant comme à la préparation 2, on a obtenu le produit recherché.
F = 202 ∼ 204°C.

### PREPARATION 5 : 4-(2,4-diméthylphényl) butylamine

En opérant comme à la préparation 2, on a obtenu le produit recherché.
F = 126 ∼ 128°C.

### PREPARATION 6 : 4-(2-méthoxyphényl) butylamine

En opérant comme à la préparation 2, on a obtenu le produit recherché.
F = 122 ∼ 124°C.

### PREPARATION 7 : 4-(4-phénoxyphényl) butylamine

En opérant comme à la préparation 2, on a obtenu le produit recherché sous forme de chlorhydrate.
F = 172 - 174°C produit que l'on transforme en amine par extraction à l'acétate d'éthyle en milieu ammoniaqué.

### PREPARATION 8 : 4-(2-phényl-1H-imidazol-1-yl) butylamine

### STADE A : N-[4-(2-phényl-1H-imidazol-1-yl) butyl] phtalimide

On ajoute en 1 heure 15 minutes à température ambiante, 4,32 g de 2-phényl imidazole en solution dans 25 cm³ de diméthyl formamide à 1,73 g d'hydrure de sodium dans 5 cm³ de diméthyl formamide. On ajoute ensuite 10,97 g de N-(4-bromobutyl) phtalimide dans 33 cm³ de diméthyl formamide et agite 48 heures à température ambiante. On extrait à l'acétate d'éthyle, sèche, évapore le solvant, chromatographie le résidu sur silice (éluant Acétate d'éthyle méthyléthylamine 95.5) reprend le résidu dans l'éther, glace, essore les cristaux, sèche et recueille 1,11 g de produit attendu F = 82-84°C.

### STADE B : 4-(2-phényl-1H-imidazol-1-yl) butylamine

On ajoute 1,6 cm³ d'hydrate d'hydrazine à 5,64 g de produit obtenu au stade A en solution dans 175 cm³ d'éthanol. On chauffe au reflux pendant 16 heures, élimine le solvant, reprend le résidu dans 25 cm³ de soude 2N et 50 cm³ d'eau, extrait à l'acétate d'éthyle, sèche, évapore le solvant, chromatographie le résidu sur silice (éluant chlorure de méthylène-méthanol-ammoniaque 9-1-0,02) et obtient 1.95 g de produit attendu - rf = 0,12.

### PREPARATION 9 : 4-(6-amino 9H-purin 9-yl) butylamine

### STADE A : N-[4-(6-amino 9H-purin 9-yl) 3-butényl] phtalimide

On ajoute 3,4 g d'hydrure de sodium à 9,6 g d'adénine dans 270 cm³ de diméthylformamide. On maintient sous agitation pendant 2 heures et demie, ajoute 20 g de N-(4-bromobutyl) phtalimide agite à température ambiante pendant 74 heures, filtre, évapore le solvant du filtrat, lave le précipité à l'éther, à l'eau, à l'éthanol puis de nouveau à l'éther, sèche. On dissout le résidu dans du méthanol à 65°C, glace et recueille 15.00 g de produit attendu.

### STADE B : 4-(6-amino 9H-purin 9-yl) butylamine

On chauffe au reflux 14,01 g de produit obtenu ci-dessus dans 728 cm³ d'éthanol et 2,02 cm³ d'hydrazine pendant 22 heures, ajoute de nouveau 2,02 cm³ d'hydrazine et poursuit la réaction pendant 11 heures. On laisse revenir à température ambiante, filtre, évapore l'éthanol, sèche et récupère 10,5 g de produit brut. On reprend 8,72 g de produit brut dans 50 cm³ de chlorure de méthylène, ajoute 9,42 cm³ d'acide trifluoroacétique, abandonne 16 heures à température ambiante, reprend le précipité à l'éther, filtre, sèche et récupère 10,24 g de produit attendu sous forme de trifluoroacétate.

### PREPARATION 10 : 4-(1H-benzimidazol-1-yl) butylamine.

En opérant comme à la préparation 8 en utilisant au départ 4,13 g de benzimidazole, on a obtenu 7,96 g du phtalimide intermédiaire (F = 136-138°C) que l'on fait réagir avec 2,5 cm³ d'hydrate d'hydrazine. On obtient 4,99 g de produit attendu que l'on transforme en oxalate à l'aide d'acide oxalique. F = 190-192°C.

### PREPARATION 11 : 2-phényl 5-(4-aminobutyl) thiazole.

En opérant comme à la préparation 9, on a obtenu le produit recherché. F ∼ 62-64°C.

### EXEMPLES DE COMPOSITIONS PHARMACEUTIQUES

On a préparé des composés renfermant :

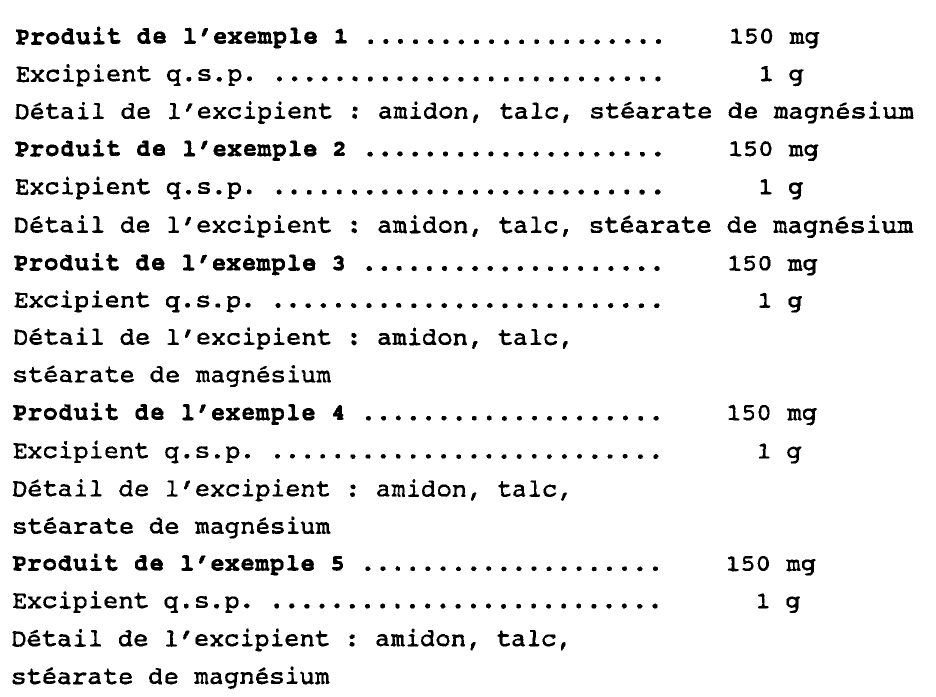

### ETUDE PHARMACOLOGIQUE DES PRODUITS DE L'INVENTION

### Méthode des dilutions en milieu liquide

On prépare une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissance est appréciée par transillumination de ce qui permet de déterminer les concentrations minimales inhibitrices (C.M.I.) exprimées en microgrammes/cm³.

Les résultats suivants ont été obtenus :

### Souches bactériennes à GRAM⁺

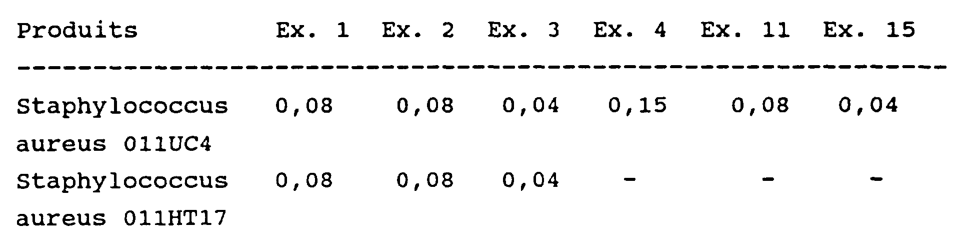

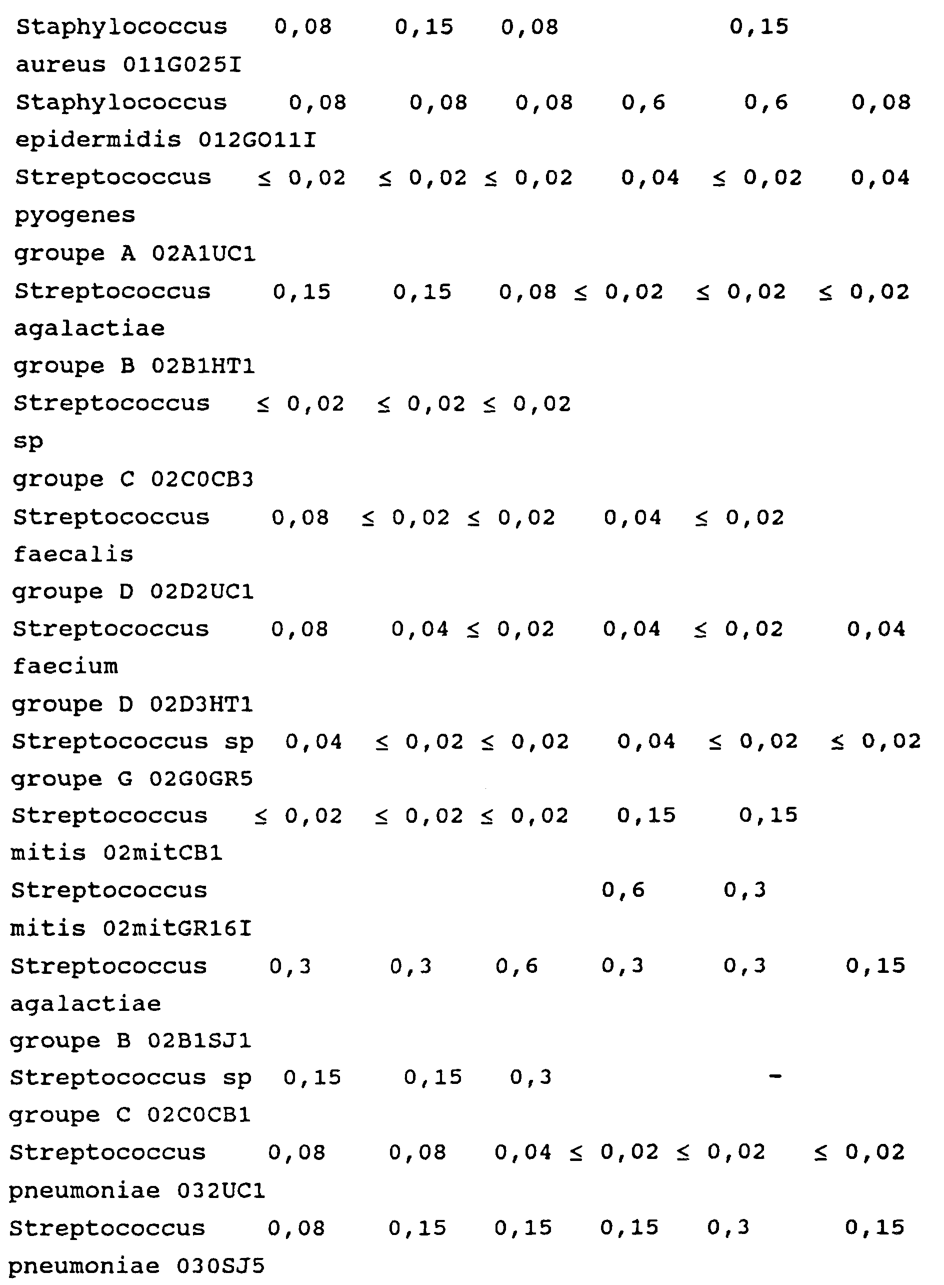

De plus, les produits des exemples 1, 2, 3, 4, 11 et 15 ont montré une activité intéressante sur les souches bactériennes à gram^{⊖} suivantes : Haemophilus Influenzae 351HT3, 351CB12, 351CA1 et 351GR6.

## Revendications

1. Les composés de formule (I) : 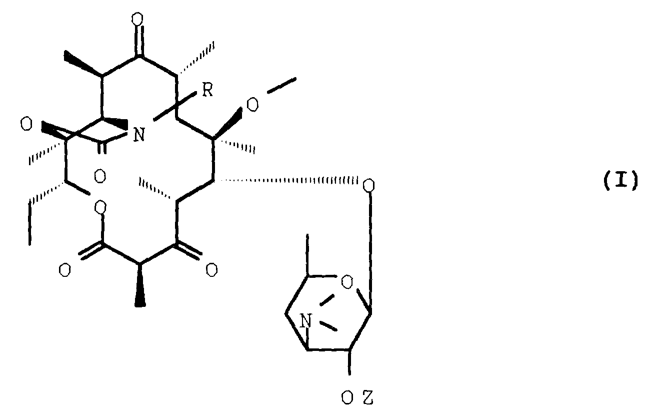 dans lesquels,
. ou bien R représente un radical (CH₂)₄ Ar₁, Ar₁ représentant :
- soit un radical aryle carbocyclique renfermant jusqu'à 18 atomes de carbone, substitué par un ou plusieurs radicaux choisis dans le groupe constitué par les atomes d'halogènes, les radicaux alkyle, linéaire, ramifié ou cyclique , alkényle et alkynyle, linéaire ou ramifié, O-alkyle, O-alkényle et O-alkynyle, S-alkyle, S-alkényle ou S-alkynyle et N-alkyle, N-alkényle et N-alkynyle, renfermant jusqu'à 12 atomes, le radical 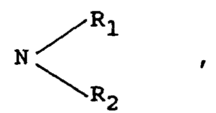 R₁ et R₂ identiques ou différents, représentant un atome d'hydrogène ou un radical alkyle renfermant jusqu'à 12 atomes de carbone, les radicaux aryle, O-aryle ou S-aryle carbocycliques ou aryle, éventuellement substitués par un ou plusieurs des substituants mentionnés ci-dessus,
- soit un radical aryle hétérocyclique comportant un ou plusieurs hétéroatomes, éventuellement substitué par un ou plusieurs des substituants mentionnés ci-dessus,
. ou bien R représente un radical X Ar₂ dans lequel X représente un radical alkyle renfermant jusqu'à 6 atomes de carbone, interrompu par un groupement 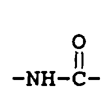 et Ar₂ représente un radical aryle ou hétéroaryle défini ci-dessus non substitué ou substitué par un ou plusieurs des substituants possibles de Ar₁ et Z représente un atome d'hydrogène ou un radical acyle renfermant jusqu'à 18 atomes de carbone ainsi que les sels d'addition avec les acides des composés de formule (I).

2. Les composés de formule (I) tels que définis à la revendication 1 dans lesquels Z représente un atome d'hydrogène.

3. Les composés de formule (I) tels que définis à la revendication 1 ou 2 dans lesquels R représente le radical (CH₂)₄Ar₁, Ar₁ étant défini comme à la revendication 1.

4. Les composés de formule (I) tels que définis à l'une quelconque des revendications 1 à 3 dans lesquels Ar₁ représente un radical phényle substitué par l'un des radicaux énumérés à la revendication 1.

5. Les composés de formule (I) tels que définis à la revendication 4 dans lesquels Ar₁ représente un radical phényle substitué par un ou plusieurs atomes d'halogène.

6. Les composés de formule (I) tels que définis à la revendication 5 dans lesquels Ar₁ représente un radical phényle substitué par un ou plusieurs atomes de chlore.

7. Les composés de formule (I) tels que définis à la revendication 6 dans lesquels Ar₁ représente un radical 4-chloro phényle.

8. Les composés de formule (I) tels que définis à la revendications 4 dans lesquels Ar₁ représente un radical phényle substitué par un ou plusieurs radicaux O-alkyle renfermant jusqu'à 4 atomes de carbone.

9. Les composés de formule (I) tels que définis à la revendication 8 dans lesquels Ar₁ représente un radical phényle substitué par un ou plusieurs radicaux méthoxy.

10. Les composés de formule (I) tels que définis à la revendication 9 dans lesquels Ar₁ représente un radical 4-méthoxyphényle.

11. Les composés de formule (I) tels que définis à la revendication 1, 2 ou 3 dans lesquels Ar₁ représente un radical aryle hétérocyclique à 5 chaînons, éventuellement substitué.

12. Les composés de formule (I) tels que définis à la revendication 11 dans lesquels Ar₁ représente un radical thiényle éventuellement substitué.

13. Les composés de formule (I) tels que définis à la revendication 12 dans lesquels Ar₁ représente un radical thiényle.

14. Les composés de formule (I) tels que définis à la revendication 11 dans lesquels Ar₁ représente un radical imidazolyle éventuellement substitué.

15. Les composés de formule (I) tels que définis à la revendication 14 dans lesquels Ar₁ représente un radical imidazolyle.

16. Les composés de formule (I) tels que définis à la revendication 1, 2 ou 3, dans lesquels Ar₁ représente un radical biphényle éventuellement substitué.

17. Les composés de formule (I) tels que définis à la revendication 16, dans lesquels Ar₁ représente un radical : 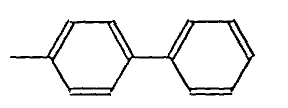 éventuellement substitué.

18. Les composés de formule (I) tels que définis à la revendication 17, dans lesquels Ar₁ représente un radical :

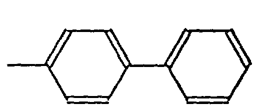

19. Les composés de formule (I) tels que définis à l'une quelconque des revendications 1 à 3, dans lesquels R représente un radical X₁ Ar₂ dans laquelle X₁ représente un radical alkyle renfermant jusqu'à 6 atomes de carbone, interrompu par un radical 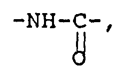 et Ar₂ conserve la même signification que précédemment.

20. Les composés de formule (I) tels que définis à la revendication 19, dans lesquels R représente un radical 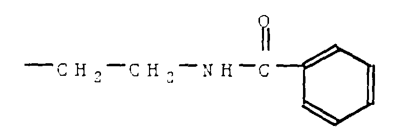 le radical phényle étant éventuellement substitué par un ou plusieurs des substituants définis à la revendication 1.

21. Les composés de formule (I) dont les noms suivent :
- 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-chlorophényl) butyl) imino)) érythromycine,
- 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-méthoxyphényl) butyl) imino)) érythromycine,
- 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(2-thiényl) butyl) imino)) érythromycine,
- 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(1,1'-biphényl) 4-yl) butyl) imino)) érythromycine,
- 11,12-didéoxy 2-de((2,6-didéoxy 3-C-méthyl 3-O-méthyl alpha-L-ribohexopyranosyl) oxy) 6-O-méthyl 3-oxo 12,11-(oxycarbonyl ((4-((1H)-imidazol-1-yl) butyl) imino)) érythromycine,
- 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((2-((phénylcarbonyl) amino) éthyl) imino)) érythromycine.

22. Les composés de formule (I) dont les noms suivent :
- le 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-quinoléiny) butyl) imino)) érythromycine
- le 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl (4-(1H-benzimidazol-1-yl)butyl) imino)) érythromycine
- le 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl (4-(2-phenyl-5-thiazolyl)butyl) imino)) érythromycine

23. A titre de médicaments, les composés de formule (I) tels que définis à l'une quelconque des revendications 1 à 22, ainsi que leurs sels d'addition avec les acides pharmaceutiquement acceptables.

24. Les compositions pharmaceutiques renfermant comme principe actif au moins un médicament défini à l'une quelconque des revendications 22 ou 23.

25. Procédé de préparation des composés de formule (I) tels que définis à la revendication 1, caractérisé en ce que l'on soumet un composé de formule (II) : 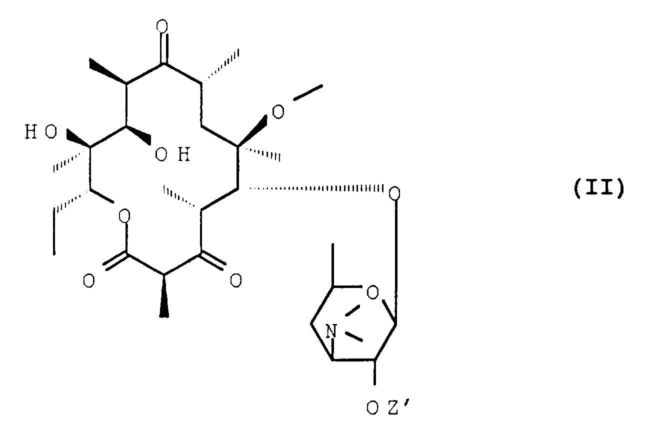 dans laquelle Z' représente le reste d'un acide carboxylique renfermant jusqu'à 18 atomes de carbone, à l'action d'un agent capable d'activer sélectivement l'hydroxyle en 11 pour obtenir le composé de formule (III) : 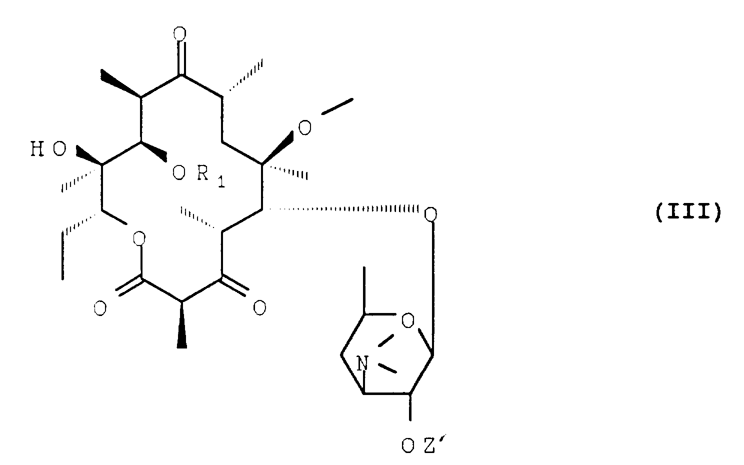 dans laquelle R₁ représente le reste d'un groupement facilement clivable, que l'on soumet à l'action d'une base, pour obtenir le composé de formule (IV) : 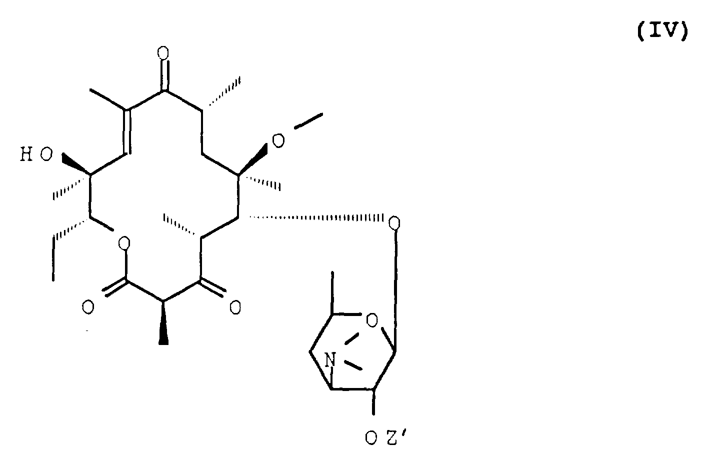 puis soumet le composé de formule (IV) :
- soit à l'action d'un composé de formule (V) :
R-N=C=O (V)
dans laquelle R est défini comme à la revendication 1, pour obtenir le composé de formule (VI) : 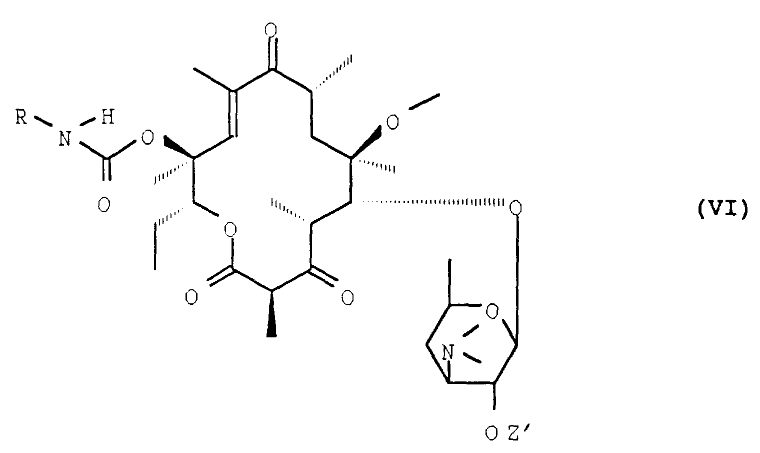 qui se cyclise soit spontanément par chauffage, soit que l'on soumet à l'action d'un agent de cyclisation pour obtenir le composé de formule (IA) : 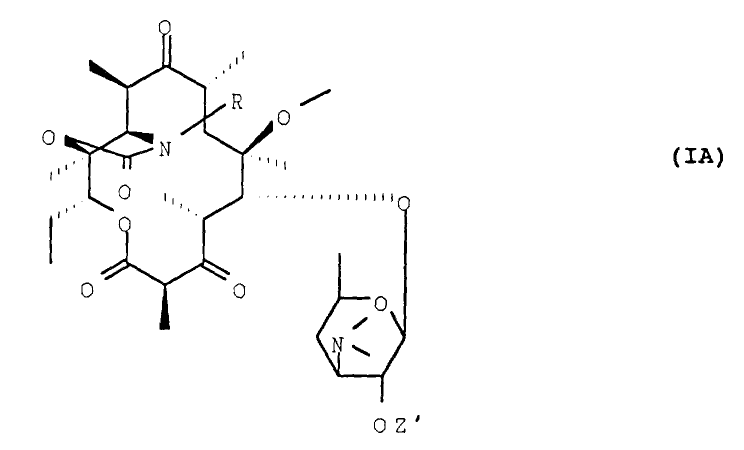 correspondant au produit de formule (I),
dans laquelle Z ne représente pas un atome d'hydrogène,
- soit à l'action du carbonyldiimidazole pour obtenir le composé de formule (VII) : 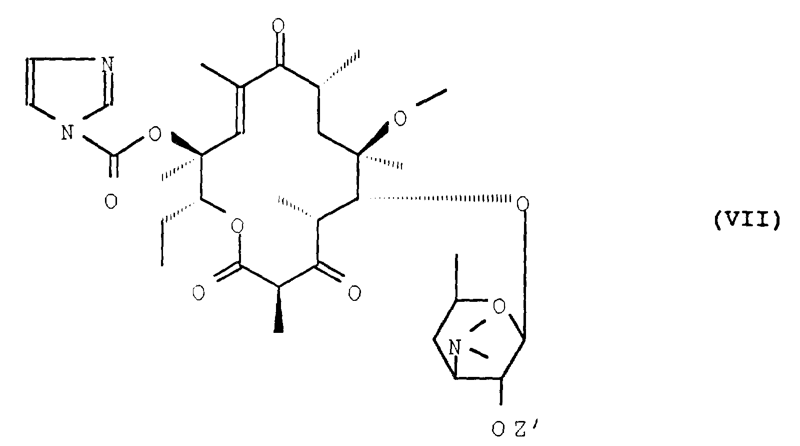 puis à l'action d'un composé de formule (VIII) :
RNH₂ (VIII)
dans laquelle R est défini comme à la revendication 1, pour obtenir le composé de formule (VI) défini précédemment qui se cyclise soit spontanément par chauffage soit que l'on soumet à l'action d'un agent de cyclisation pour obtenir le composé de formule (IA) correspondant, puis soumet le cas échéant, le composé de formule (IA) à l'action d'un agent de libération de la fonction hydroxyle en 2' et/ou le cas échéant, à l'action d'un acide pour en former le sel.

26. A titre de produits chimiques nouveaux, les composés de formules (III), (IV), (VI) et (VII).

27. A titre de produits chimiques nouveaux, tels que définis à la revendication 25, le 2'-acétate de 11-déoxy 10,11-didéhydro 3-de((2,6-didéoxy 3-C-méthyl 3-O-méthyl alpha-L-ribohexopyranosyl) oxy) 6-O-méthyl 3-oxo érythromycine et le composé 2'-hydroxy correspondant.

28. A titre de produits chimiques nouveaux, les composés de formule (VIII) telle que définie à la revendication 25, suivants :
la 4-(2-thiényl) butylamine,
la 4-(1,1'biphényl) butylamine,
la 4-(4-méthylphényl) butylamine,
la 4-(2,4-diméthylphényl) butylamine,
la 4-(2,4,6-triméthylphényl) butylamine,
la 4-(2-méthoxyphényl) butylamine.

## Patentansprüche

1. Verbindungen der Formel (I) 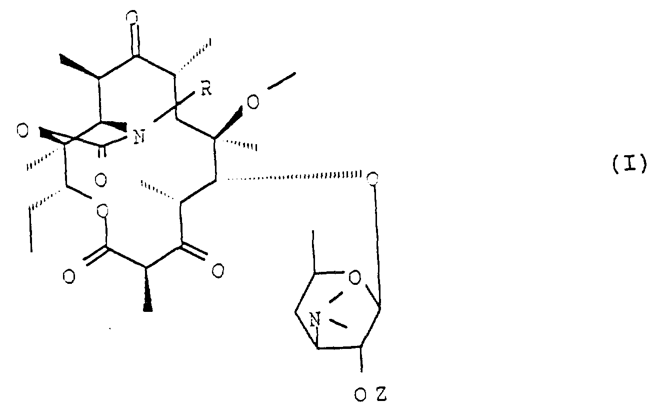 worin
. entweder R für einen Rest (CH₂)₄ Ar₁ steht, worin Ar₁ bedeutet:
- entweder einen carbocyclischen Arylrest mit bis zu 18 Kohlenstoffatomen, substituiert durch einen oder mehrere Reste, ausgewählt unter den Halogenatomen, den linearen, verzweigten oder cyclischen Alkyl-, linearen oder verzweigten Alkenyl- und Alkinyl-, O-Alkyl-, O-Alkenyl- und O-Alkinyl-, S-Alkyl-, S-Alkenyl- oder S-Alkinyl- und N-Alkyl-, N-Alkenyl-und N-Alkinylresten mit bis zu 12 Kohlenstoffatomen, dem Rest 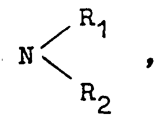 worin R₁ und R₂, identisch oder verschieden, ein Wasserstoffatom oder einen Alkylrest mit bis zu 12 Kohlenstoffatomen darstellen, den carbocyclischen Aryl-, O-Aryl- oder S-Arylresten oder Arylresten, die gegebenenfalls durch einen oder mehrere der vorstehend erwähnten Substituenten substituiert sind,
- oder einen heterocyclischen Arylrest mit einem oder mehreren Heteroatomen, der gegebenenfalls durch einen oder mehrere der vorstehend genannten Substituenten substituiert ist,
. oder R für einen Rest X Ar₂ steht, worin X einen Alkylrest mit bis zu 6 Kohlenstoffatomen, der durch eine Gruppe 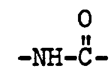 unterbrochen ist, bedeutet und Ar₂ für einen vorstehend definierten Aryl- oder Heteroarylrest, der unsubstituiert oder substituiert ist durch einen oder mehrere der möglichen Substituenten von Ar₁, steht,und Z ein Wasserstoffatom oder einen Acylrest mit bis zu 18 Kohlenstoffatomen bedeutet, sowie die Additionssalze der Verbindungen der Formel (I) mit Säuren.

2. Verbindungen der Formel (I), wie in Anspruch 1 definiert, worin Z ein Wasserstoffatom bedeutet.

3. Verbindungen der Formel (I), wie in Anspruch 1 oder 2 definiert, worin R den Rest (CH₂)₄Ar₁ wiedergibt, worin Ar₁ wie in Anspruch 1 definiert ist.

4. Verbindungen der Formel (I), wie in einem der Ansprüche 1 bis 3 definiert, worin Ar₁ einen Phenylrest bedeutet, der durch einen der in Anspruch 1 angegebenen Substituenten substituiert ist.

5. Verbindungen der Formel (I), wie in Anspruch 4 definiert, worin Ar₁ einen durch ein oder mehrere Halogenatome substituierten Phenylrest bedeutet.

6. Verbindungen der Formel (I), wie in Anspruch 5 definiert, worin Ar₁ einen Phenylrest bedeutet, der durch ein oder mehrere Chloratome substituiert ist.

7. Verbindungen der Formel (I), wie in Anspruch 6 definiert, worin Ar₁ einen 4-Chlorphenylrest bedeutet.

8. Verbindungen der Formel (I), wie in Anspruch 4 definiert, worin Ar₁ einen Phenylrest bedeutet, der durch einen oder mehrere O-Alkylreste mit bis zu 4 Kohlenstoffatomen substituiert ist.

9. Verbindungen der Formel (I), wie in Anspruch 8 definiert, worin Ar₁ einen Phenylrest bedeutet, der durch einen oder mehrere Methoxyreste substituiert ist.

10. Verbindungen der Formel (I), wie in Anspruch 9 definiert, worin Ar₁ einen 4-Methoxyphenylrest bedeutet.

11. Verbindungen der Formel (I), wie in Anspruch 1, 2 oder 3 definiert, worin Ar₁ einen heterocyclischen Arylrest mit 5 Ringgliedern bedeutet, der gegebenenfalls substituiert ist.

12. Verbindungen der Formel (I), wie in Anspruch 11 definiert, worin Ar₁ einen gegebenenfalls substituierten Thienylrest bedeutet.

13. Verbindungen der Formel (I), wie in Anspruch 12 definiert, worin Ar₁ einen Thienylrest bedeutet.

14. Verbindungen der Formel (I), wie in Anspruch 11 definiert, worin Ar₁ einen gegebenenfalls substituierten Imidazolylrest bedeutet.

15. Verbindungen der Formel (I), wie in Anspruch 14 definiert, worin Ar₁ einen Imidazolylrest bedeutet.

16. Verbindungen der Formel (I), wie in Anspruch 1, 2 oder 3 definiert, worin Ar₁ einen gegebenenfalls substituierten Biphenylrest bedeutet.

17. Verbindungen der Formel (I), wie in Anspruch 16 definiert, worin Ar₁ einen Rest 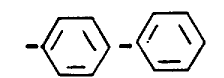 bedeutet, der gegebenenfalls substituiert ist.

18. Verbindungen der Formel (I), wie in Anspruch 17 definiert, worin Ar₁ einen Rest 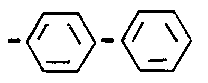 bedeutet.

19. Verbindungen der Formel (I), wie in einem der Ansprüche 1 bis 3 definiert, worin R einen Rest X₁ Ar₂ bedeutet, in dem X₁ einen Alkylrest mit bis zu 6 Kohlenstoffatomen darstellt, der durch einen Rest 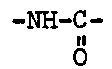 unterbrochen ist, und Ar₂ die vorstehend angegebene Bedeutung besitzt.

20. Verbindungen der Formel (I), wie in Anspruch 19 definiert, worin R einen Rest 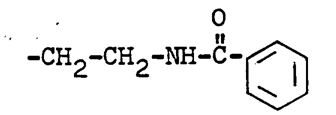 bedeutet, wobei der Phenylrest gegebenenfalls durch einen oder mehrere der in Anspruch 1 definierten Substituenten substituiert ist.

21. Verbindungen der Formel (I) mit den folgenden Bezeichnungen:
11,12-Didesoxy-3-de-[(2,6-didesoxy-3-C-methyl-3-O-methyl-α-L-ribohexopyranosyl)-oxyl-6-O-methyl-3-oxo-12,11-[oxycarbonyl-[[4-(4-chlorphenyl)-butyl]-imino ]]-erythromycin,
11,12-Didesoxy-5-de-[(2,6-didesoxy-3-C-methyl-3-O-methyl-α-L-ribohexopyranosyl)-oxy]-6-O-methyl-3-oxo-12,11-[oxycarbonyl-[[4-(4-methoxyphenyl)-butyl]-imino]]-erythromycin,
11,12-Didesoxy-3-de-[(2,6-didesoxy-3-C-methyl-3-O-methyl-α-L-ribohexopyranosyl)-oxyl-6-O-methyl-3-oxo-12,11-[oxycarbonyl-[[4-(2-thienyl)-butyl]-imino]]-erythromycin,
11,12-Didesoxy-3-de-[(2,6-didesoxy-3-C-methyl-3-O-methyl-α-L-ribohexopyranosyl)-oxy]-6-O-methyl-3-oxo-12,11-[oxycarbonyl-[[4-(1,1'-biphenyl)-4-yl]-butyl]-imino]]-erythromycin,
11,12-Didesoxy-2-de-[(2,6-didesoxy-3-C-methyl-3-O-methyl-α-L-ribohexopyranosyl)-oxy]-6-O-methyl-3-oxo-12,11-[oxycarbonyl-[[4-[(1H)-imidazol-1-yl]-butyl]-imino]]erythromycin,
11,12-Didesoxy-3-de-[(2,6-didesoxy-3-C-methyl-3-O-methyl-α-L-ribohexopyranosyl)-oxyl-6-O-methyl-3-oxo-12,11-[oxycarbonyl-[[2-[(phenylcarbonyl)-amino]-ethyl]-imino]]-erythromycin.

22. Verbindungen der Formel (I) mit den folgenden Bezeichnungen:
11,12-Didesoxy-3-de-[(2,6-didesoxy-3-C-methyl-3-O-methyl-α-L-ribohexopyranosyl)-oxy]-6-O-methyl-3-oxo-12,11-[oxycarbonyl-[[4-(4-chinolin)-butyl]-imino]]-erythromycin,
11,12-Didesozy-3-de-[(2,6-didesoxy-3-C-methyl-3-O-methyl-α-L-ribohexopyranosyl)-oxy]-6-O-methyl-3-oxo-12,11-[oxycarbonyl-[4-(1H-benzimidazol-1-yl)-butyl]-imino]]-erythromycin,
11,12-Didesoxy-3-de-[(2,6-didesoxy-3-C-methyl-3-O-methyl-α-L-ribohexopyranosyl)-oxyl-6-O-methyl-3-oxo-12,11-[oxycarbonyl-[4-(2-phenyl-5-thiazolyl)-butyl]-imino]]-erythromycin.

23. Als Arzneimittel die Verbindungen der Formel (I), wie in einem der Ansprüche 1 bis 22 definiert, sowie deren Additionssalze mit pharmazeutisch annehmbaren Säuren.

24. Pharmazeutische Zusammensetzungen, enthaltend als Wirkstoff zumindest ein Arzneimittel, wie in einem der Ansprüche 22 oder 23 definiert.

25. Verfahren zur Herstellung der Verbindungen der Formel (I), wie in Anspruch 1 definiert, dadurch gekennzeichnet, daß man eine Verbindung der Formel (II) 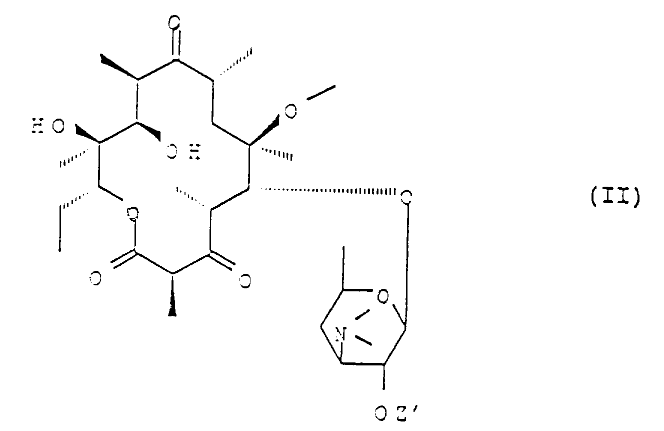 worin Z' den Rest einer Carbonsäure mit bis zu 18 Kohlenstoffatomen bedeutet, der Einwirkung eines Mittels unterzieht, das in der Lage ist, selektiv die Hydroxylgruppe in 11-Stellung zu aktivieren, um zu der Verbindung der Formel (III) 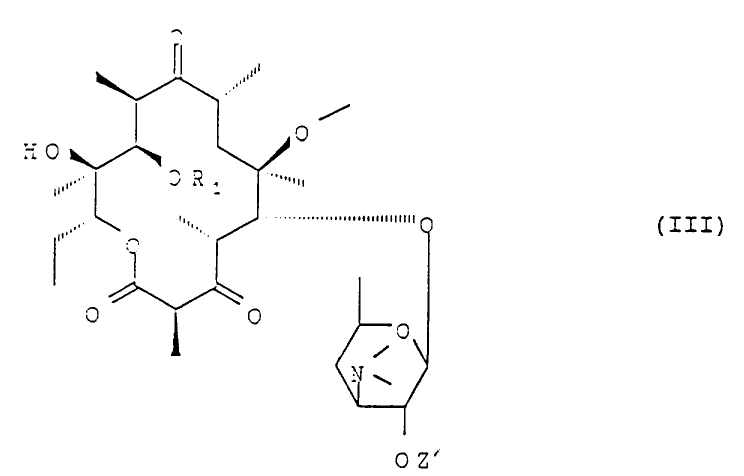 worin R₁ den Rest einer leicht abspaltbaren Gruppe bedeutet, zu gelangen, welche man der Einwirkung einer Base unterzieht, um zu der Verbindung der Formel (IV) 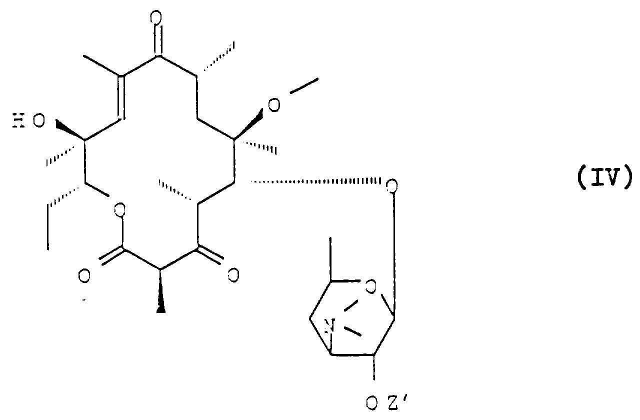 zu gelangen, wonach man die Verbindung der Formel (IV)
- entweder der Einwirkung einer Verbindung der Formel (V)
R-N=C=O (V)
unterzieht, worin R wie in Anspruch 1 definiert ist, um zu der Verbindung der Formel (VI) 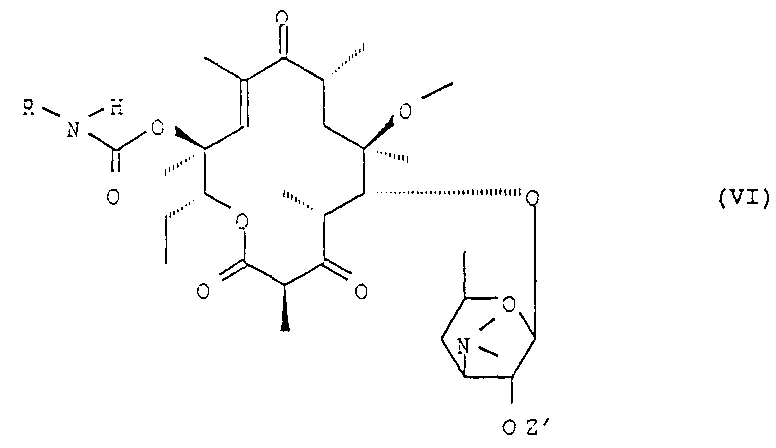 zu gelangen, die durch Erhitzen spontan cyclisiert,oder die man der Einwirkung eines Cyclisierungsmittels unterzieht, um zu der Verbindung der Formel (IA) 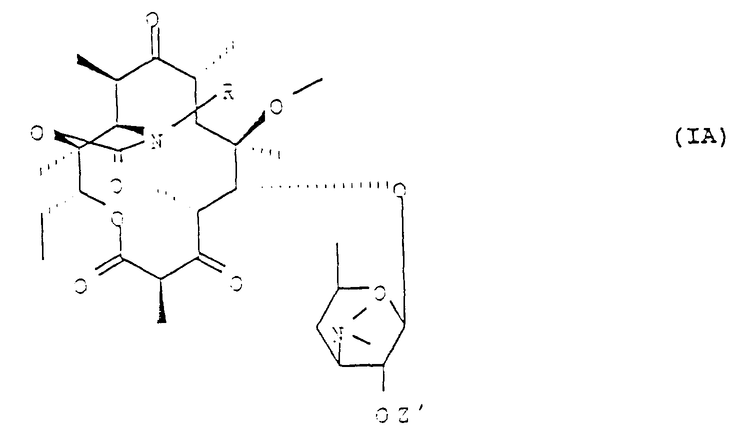 entsprechend dem Produkt der Formel (I), worin Z kein Wasserstoffatom bedeutet, zu gelangen,
- oder der Einwirkung von Carbonyldiimidazol unterzieht, um zu der Verbindung der Formel (VII) 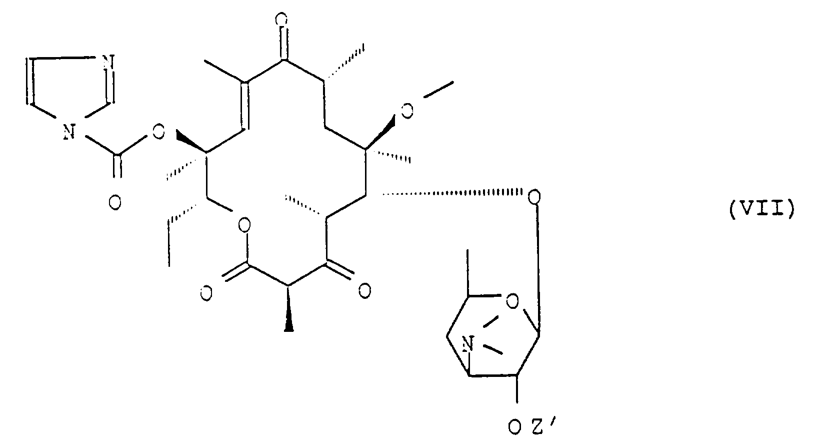 zu gelangen, hiernach der Einwirkung einer Verbindung der Formel (VIII)
RNH₂ (VIII)
unterzieht, worin R wie in Anspruch 1 definiert ist, um zu der Verbindung der Formel (VI), wie vorstehend definiert, zu gelangen, die spontan durch Erhitzen cyclisiert, oder die man der Einwirkung eines Cyclisierungsmittels unterzieht, um zu der entsprechenden Verbindung der Formel (IA) zu gelangen, hiernach gegebenenfalls die Verbindung der Formel (IA) der Einwirkung eines Mittels für die Freisetzung der Hydroxylfunktion in 2'-Stellung und/oder gegebenenfalls der Einwirkung einer Säure unterzieht, um hieraus das Salz zu bilden.

26. Als neue chemische Produkte die Verbindungen der Formeln (III), (IV), (VI) und (VII).

27. Als neue chemische Produkte, wie in Anspruch 25 definiert, das 2'-Acetat von 11-Desoxy-10,11-didehydro-3-de[(2,6-didesoxy-3-C-methyl-3-O-methyl-α-L-ribohexopyranosyl)oxy]-6-O-methyl-3-oxo-erythromycin und die entsprechende 2'-Hydroxyverbindung.

28. Als neue chemische Produkte die Verbindungen der Formel (VIII), wie in Anspruch 25 definiert, mit den folgenden Bezeichnungen:
4-(2-Thienyl)-butylamin,
4-(1,1'-Biphenyl)-butylamin,
4-(4-Methylphenyl)-butylamin,
4-(2,4-Dimethylphenyl)-butylamin,
4-(2,4,6-Trimethylphenyl)-butylamin,
4-(2-Methoxyphenyl)-butylamin.

## Claims

1. The compounds of formula (I): 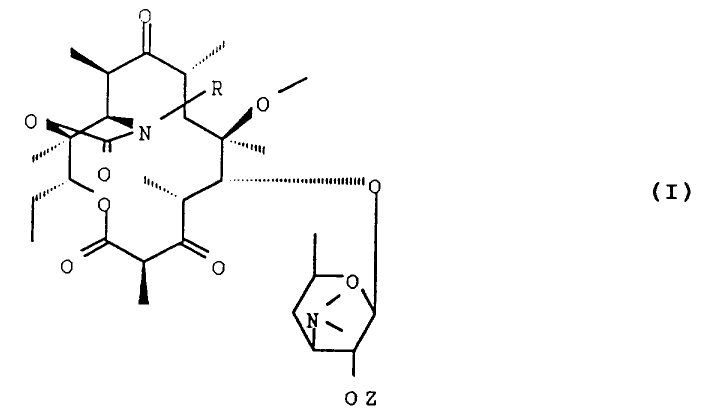 in which,
. either R represents a (CH₂)ₙAr₁ radical, Ar₁ representing:
- either a carbocyclic aryl radical containing up to 18 carbon atoms, substituted by one or more radicals chosen from the group constituted by halogen atoms, linear, branched or cyclic alkyl radicals, linear or branched alkenyl and alkynyl radicals, O-alkyl, O-alkenyl and O-alkynyl, S-alkyl, S-alkenyl or S-alkynyl and N-alkyl, N-alkenyl and N-alkynyl radicals, containing up to 12 carbon atoms, the 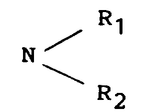 radical, R₁ and R₂ being identical or different, representing a hydrogen atom or an alkyl radical containing up to 12 carbon atoms, the carbocyclic aryl, O-aryl or S-aryl or aryl radicals, optionally substituted by one or more of the substituents mentioned above,
- or a heterocyclic aryl radical containing one or more heteroatoms, optionally substituted by one or more of the substituents mentioned above,
. or R represents an X Ar₂ radical in which X represents an alkyl radical containing up to 6 carbon atoms, interupted by an 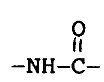 group, and Ar₂ represents an aryl or heteroaryl radical defined above, non-substituted or substituted by one or more of the possible substituents of Ar₁ and Z represents a hydrogen atom or an acyl radical containing up to 18 carbon atoms as well as the addition salts with acids of the compounds of formula (I).

2. The compounds of formula (I) as defined in claim 1 in which Z represents a hydrogen atom.

3. The compounds of formula (I) as defined in claim 1 or 2 in which R represents the (CH₂)₄Ar₁ radical, Ar₁ being defined as in claim 1.

4. The compounds of formula (I) as defined in any one of claims 1 to 3 in which Ar₁ represents a phenyl radical substituted by one of the radicals enumerated in claim 1.

5. The compounds of formula (I) as defined in claim 4 in which Ar₁ represents a phenyl radical substituted by one or more halogen atoms.

6. The compounds of formula (I) as defined in claim 5 in which Ar₁ represents a phenyl radical substituted by one or more chlorine atoms.

7. The compounds of formula (I) as defined in claim 6 in which Ar₁ represents a 4-chloro phenyl radical.

8. The compounds of formula (I) as defined in claim 4 in which Ar₁ represents a phenyl radical substituted by one or more O-alkyl radicals containing up to 4 carbon atoms.

9. The compounds of formula (I) as defined in claim 8 in which Ar₁ represents a phenyl radical substituted by one or more methoxy radicals.

10. The compounds of formula (I) as defined in claim 9 in which Ar₁ represents a 4-methoxyphenyl radical.

11. The compounds of formula (I) as defined in claim 1, 2 or 3 in which Ar₁ represents an optionally substituted heterocyclic aryl radical with 5 members.

12. The compounds of formula (I) as defined in claim 11 in which Ar₁ represents an optionally substituted thienyl radical.

13. The compounds of formula (I) as defined in claim 12 in which Ar₁ represents a thienyl radical.

14. The compounds of formula (I) as defined in claim 11 in which Ar₁ represents an optionally substituted imidazolyl radical.

15. The compounds of formula (I) as defined in claim 14 in which Ar₁ represents an imidazolyl radical.

16. The compounds of formula (I) as defined in claim 1, 2 or 3, in which Ar₁ represents an optionally substituted biphenyl radical.

17. The compounds of formula (I) as defined in claim 16, in which Ar₁ represents an optionally substituted: 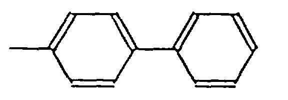 radical.

18. The compounds of formula (I) as defined in claim 17, in which Ar₁ represents a: 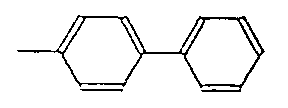 radical.

19. The compounds of formula (I) as defined in any one of claims 1 to 3, in which R represents an X₁ Ar₂ radical in which X₁ represents an alkyl radical containing up to 6 carbon atoms, interrupted by an 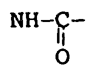 radical, and Ar₂ retains the same meaning as previously.

20. The compounds of formula (I) as defined in claim 19, in which R represents a: 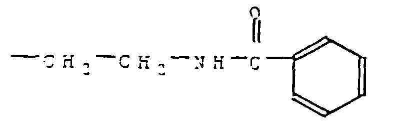 radical.
the phenyl radical being optionally substituted by one or more of the substituents defined in claim 1.

21. The compounds of formula (I) whose names follow:
- 11,12-dideoxy 3-de((2,6-dideoxy 3-C-methyl 3-O-methyl alpha-L-ribohexopyranosyl) oxy) 6-O-methyl 3-oxo 12,11-(oxycarbonyl ((4-(4-chlorophenyl) butyl) imino)) erythromycin,
- 11,12-dideoxy 3-de((2,6-dideoxy 3-C-methyl 3-O-methyl alpha-L-ribohexopyranosyl) oxy) 6-O-methyl 3-oxo 12,11-(oxycarbonyl ((4-(4-methoxyphenyl) butyl) imino)) erythromycin,
- 11,12-dideoxy 3-de((2,6-dideoxy 3-C-methyl 3-O-methyl alpha-L-ribohexopyranosyl) oxy) 6-O-methyl 3-oxo 12,11-(oxycarbonyl ((4-(2-thienyl) butyl) imino)) erythromycin,
- 11,12-dideoxy 3-de((2,6-dideoxy 3-C-methyl 3-O-methyl alpha-L-ribohexopyranosyl) oxy) 6-O-methyl 3-oxo 12,11-(oxycarbonyl ((4-(1,1'-biphenyl) 4-yl) butyl) imino)) erythromycin,
- 11,12-dideoxy 2-de((2,6-dideoxy 3-C-methyl 3-O-methyl alpha-L-ribohexopyranosyl) oxy) 6-O-methyl 3-oxo 12,11-(oxycarbonyl ((4-((1H)-imidazol-1-yl) butyl) imino)) erythromycin,
- 11,12-dideoxy 3-de((2,6-dideoxy 3-C-methyl 3-O-methyl alpha-L-ribohexopyranosyl) oxy) 6-O-methyl 3-oxo 12,11-(oxycarbonyl ((2-((phenylcarbonyl) amino) ethyl) imino)) erythromycin.

22. The compounds of formula (I) whose names follow:
- 11,12-dideoxy 3-de((2,6-dideoxy 3-C-methyl 3-O-methyl alpha-L-ribohexopyranosyl) oxy) 6-O-methyl 3-oxo 12,11-(oxycarbonyl ((4-(4-quinoliny) butyl) imino)) erythromycin,
- 11,12-dideoxy 3-de((2,6-dideoxy 3-C-methyl 3-O-methyl alpha-L-ribohexopyranosyl) oxy) 6-O-methyl 3-oxo 12,11-(oxycarbonyl ((4-(1H-benzimidazol-1-yl)butyl) imino)) erythromycin,
- 11,12-dideoxy 3-de((2,6-dideoxy 3-C-methyl 3-O-methyl alpha-L-ribohexopyranosyl) oxy) 6-O-methyl 3-oxo 12,11-(oxycarbonyl ((4-(2-phenyl-5-thiazolyl)butyl) imino)) erythromycin,

23. As medicaments, the compounds of formula (I) as defined in any one of claims 1 to 22, as well as their addition salts with pharmaceutically acceptable acids.

24. The pharmaceutical compositions containing as active ingredient at least one medicament defined in any one of claims 22 or 23.

25. Preparation process for the compounds of formula (I) as defined in claim 1, characterized in that a compound of formula (II): 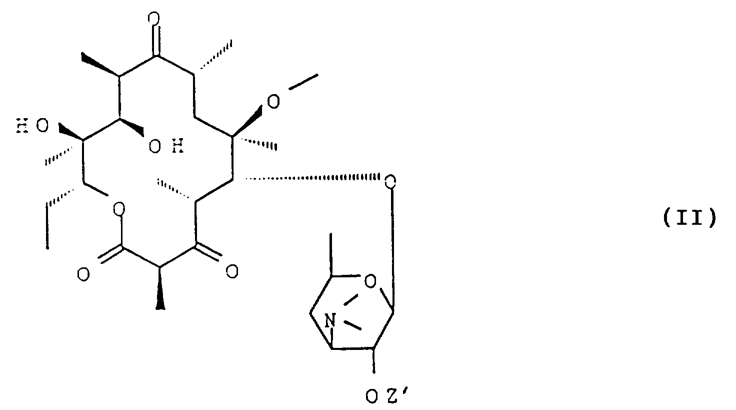 in which Z' represents the remainder of a carboxylic acid containing up to 18 carbon atoms, is subjected to the action of an agent capable of selectively activating the hydroxyl in position 11 in order to obtain the compound of formula (III): 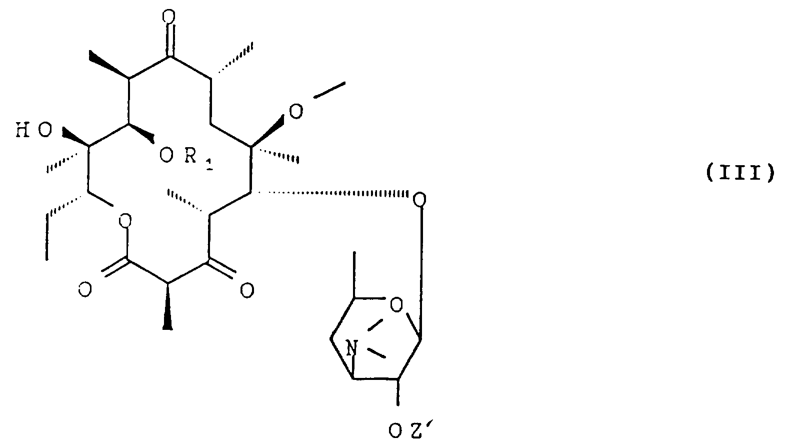 in which R₁ represents the remainder of an easily cleavable group, which is subjected to the action of a base, in order to obtain the compound of formula (IV): 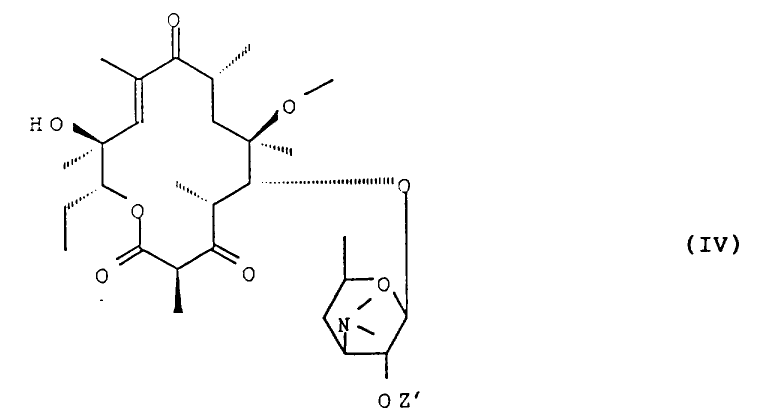 then the compound of formula (IV) is subjected:
- either to the action of a compound of formula (V):
R-N=C=O (V)
in which R is defined as in claim 1, in order to obtain the compound of formula (VI): 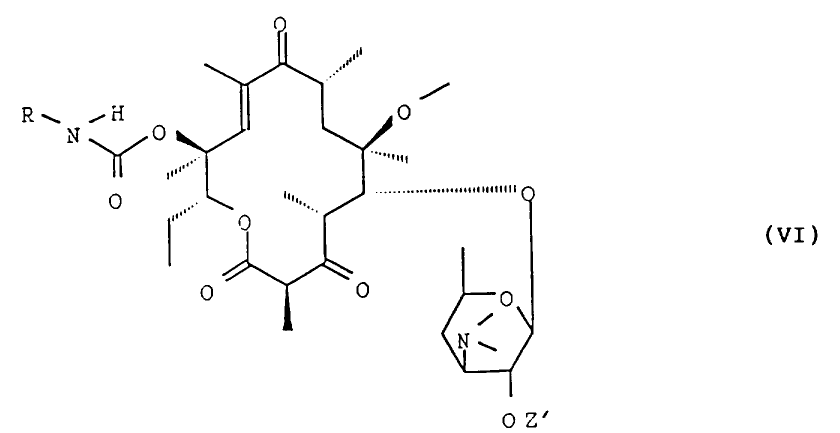 which is cyclized either spontaneously by heating, or which is subjected to the action of a cyclization agent in order to obtain the compound of formula (IA): 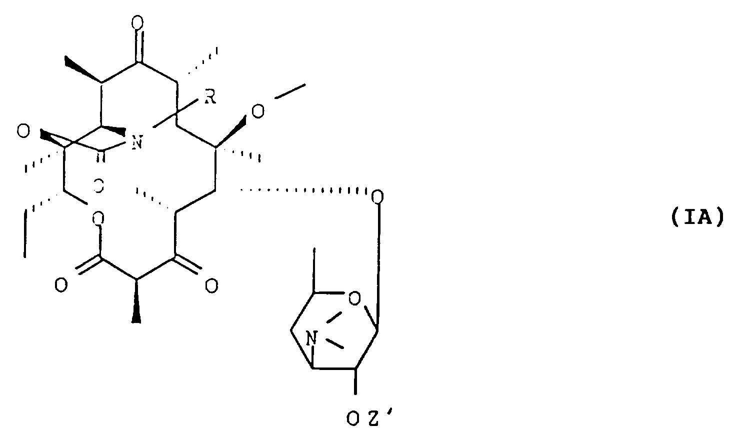 corresponding to the product of formula (I),
in which Z does not represent a hydrogen atom,
- or to the action of carbonyldiimidazole in order to obtain the compound of formula (VII): 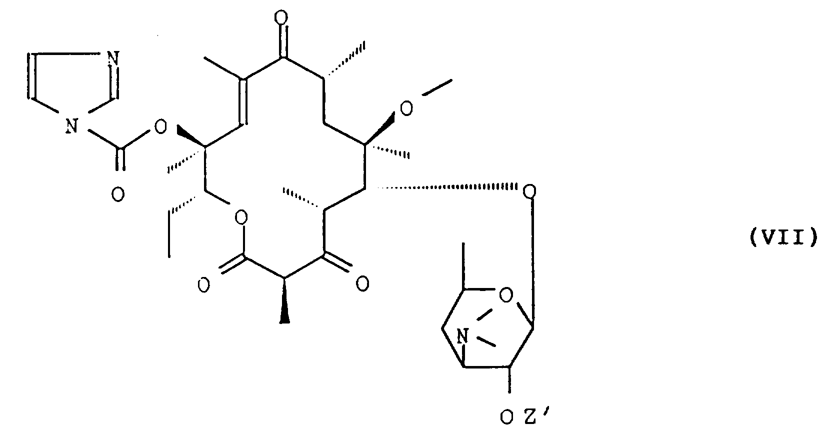 then to the action of a compound of formula (VIII):
RNH₂ (VIII)
in which R is defined as in claim 1, in order to obtain the compound of formula (VI) defined previously which is cyclized either spontaneously by heating or which is subjected to the action of a cyclization agent in order to obtain the corresponding compound of formula (IA), then if appropriate the compound of formula (IA) is subjected to the action of an agent which releases the hydroxyl function in position 2' and/or if appropriate, to the action of an acid in order to form the salt.

26. As new chemical products, the compounds of formulae (III), (IV), (VI) and (VII).

27. As new chemical products, as defined in claim 25, 11-deoxy 10,11-didehydro 3-de((2,6-dideoxy 3-C-methyl 3-O-methyl alpha-L-ribohexopyranosyl) oxy) 6-O-methyl 3-oxo erythromycin 2'-acetate and the corresponding 2'-hydroxy compound.

28. As new chemical products, the following compounds of formula (VIII) as defined in claim 25:
4-(2-thienyl) butylamine,
4-(1,1'biphenyl) butylamine,
4-(4-methylphenyl) butylamine,
4-(2,4-dimethylphenyl) butylamine,
4-(2,4,6-trimethylphenyl) butylamine,
4-(2-methoxyphenyl) butylamine.
